# EUROPEAN PATENT SPECIFICATION

(11) **EP 4 448 107 B1**
(45) Date of publication and mention of the grant of the patent: **05.11.2025**
(21) Application number: 22843870.1
(22) Date of filing: 13.12.2022
(51) Int. Cl.: A61P 33/06, A61P 35/00, C07D 323/00, A61K 31/357

(54) **DRUG DELIVERY SYSTEMS BASED ON ENDOPEROXIDES USEFUL IN DIAGNOSIS AND THERAPY, AND METHODS THEREOF**
ARZNEIMITTELABGABESYSTEME AUF BASIS VON ENDOPEROXIDEN ZUR DIAGNOSE UND THERAPIE SOWIE VERFAHREN DAFÜR
SYSTÈMES D'ADMINISTRATION DE MÉDICAMENT À BASE D'ENDOPEROXYDES UTILES DANS LE DIAGNOSTIC ET LA THÉRAPIE, ET LEURS PROCÉDÉS

(30) Priority: 16.12.2021 PT 2021117644
(43) Date of publication of application: 23.10.2024
(73) Proprietor: Faculdade de Farmácia da Universidade de Lisboa, 1649-003 Lisboa (PT)
(72) Inventor: SILVA MAGALHÃES, Diogo, 1950-074 Lisboa (PT); MOREIRA, Rui, 1750-447 Lisboa (PT); LOPES, Francisca, 1750-447 LISBOA (PT); RODRIGUES, Cecília, 1990-291 LISBOA (PT); MARQUES, Vanda, 2805-353 ALMADA (PT)
(74) Representative: Gata-Goncalves, Ligia
(86) International application number: PCT/IB2022/062107
(87) International publication number: WO 2023/111829

(56) References cited:
- WO-A1-2008/038299
- WO-A1-2020/240266
- MENDES ANDREIA ET AL: "1,2,4-Trioxolane and 1,2,4,5-Tetraoxane Endoperoxides against Old-World Leishmania Parasites: In Vitro Activity and Mode of Action", PHARMACEUTICALS, vol. 15, no. 4, 3 April 2022 (2022-04-03), pages 446, XP093030341, DOI: 10.3390/ph15040446
- MIRANDA DANIELA ET AL: "Novel Endoperoxide-Based Transmission-Blocking Antimalarials with Liver- and Blood-Schizontocidal Activities", ACS MEDICINAL CHEMISTRY LETTERS, vol. 5, no. 2, 23 December 2013 (2013-12-23), US, pages 108 - 112, XP055953650, ISSN: 1948-5875, DOI: 10.1021/ml4002985
- YANG JING-JING ET AL: "Design, synthesis and biological evaluation of praziquantel and endoperoxide conjugates as antischistosomal agents", FUTURE MEDICINAL CHEMISTRY, vol. 7, no. 6, 1 April 2015 (2015-04-01), GB, pages 713 - 725, XP093029987, ISSN: 1756-8919, Retrieved from the Internet <URL:http://dx.doi.org/10.4155/fmc.15.20> DOI: 10.4155/fmc.15.20
- AMEWU RICHARD K ET AL: "Synthesis and evaluation of the antimalarial, anticancer, and caspase 3 activities of tetraoxane dimers", BIOORGANIC & MEDICINAL CHEMISTRY, ELSEVIER, AMSTERDAM, NL, vol. 21, no. 23, 1 October 2013 (2013-10-01), pages 7392 - 7397, XP028760010, ISSN: 0968-0896, DOI: 10.1016/J.BMC.2013.09.047
- ANTOLÍNEZ ISABEL V. ET AL: "Tetroxanes as New Agents against Leishmania amazonensis", CHEMISTRY & BIODIVERSITY, vol. 17, no. 6, 1 June 2020 (2020-06-01), CH, XP093030116, ISSN: 1612-1872, Retrieved from the Internet <URL:https://onlinelibrary.wiley.com/doi/full-xml/10.1002/cbdv.202000142> DOI: 10.1002/cbdv.202000142
- O'NEILL P M ET AL: "Co(thd)"2: a superior catalyst for aerobic epoxidation and hydroperoxysilylation of unactivated alkenes: application to the synthesis of spiro-1,2,4-trioxanes", TETRAHEDRON LETTERS, ELSEVIER, AMSTERDAM , NL, vol. 44, no. 44, 27 October 2003 (2003-10-27), pages 8135 - 8138, XP004461070, ISSN: 0040-4039, DOI: 10.1016/J.TETLET.2003.09.033
- RODE AMBADAS B. ET AL: "Synthesis and Evaluation of Stearic Acid Derivatives as Cetane Number Improvers", BULLETIN OF THE KOREAN CHEMICAL SOCIETY, vol. 32, no. 6, 20 June 2011 (2011-06-20), KR, pages 1965 - 1969, XP093030145, ISSN: 0253-2964, Retrieved from the Internet <URL:http://koreascience.or.kr/article/JAKO201120956422880.pdf> DOI: 10.5012/bkcs.2011.32.6.1965

## Description

### Technical domain of the invention

The present invention relates to novel drug delivery systems based on endoperoxide moieties such as 1,2,4,5-tetraoxanes, namely compounds of formula I, appropriately modified to release active pharmaceutical ingredients (API) in the presence of higher levels of Fe(II), in a subject, 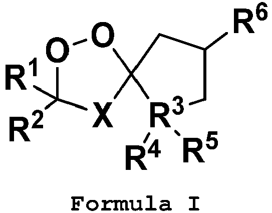 notably the derivative compounds including enantiomers and racemic mixtures thereof with the following formulae IIa, IIb, IIc, IId, IIe: 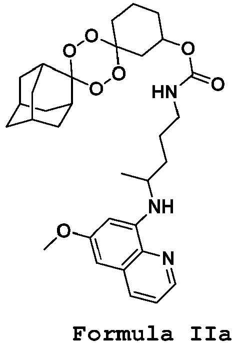 **Wherein:**
**R¹** is adamantyl
**R²** is adamantyl
**X** is -O-O-
**R³** is -CH(R⁴)-CH(R⁵)-
**R⁴** is hydrogen
**R⁵** is (4-((7-methoxyquinolin-5-yl)amino)pentyl) carbamoyloxy and
**R⁶** is hydrogen; 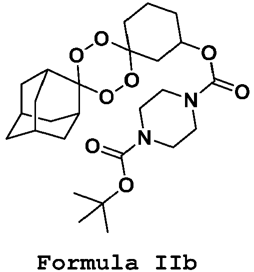 **Wherein:**
   **R¹** is adamantyl
   **R²** is adamantyl
   **X** is -O-O-
   **R³** is -CH(R⁴)-CH(R⁵)-
   **R⁴** is hydrogen
   **R⁵** is 1-(tert-butyl) piperazine-1,4-dicarboxylate and
   **R⁶** is hydrogen; 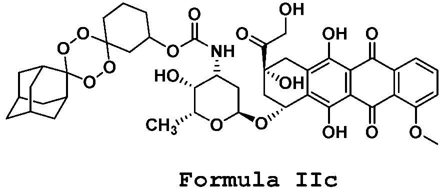 **wherein:**
      **R¹** is adamantyl
      **R²** is adamantyl
      **X** is -O-O-
      **R³** is -CH(R⁴)-CH(R⁵)-
      **R⁴** is hydrogen
      **R⁵** is (3-hydroxy-2-methyl-6-((2S,4S)-4,5,12-trihydroxy-4-(2- hydroxyacetyl)-10-methoxy-6,11-dioxo-1,2,3,4,6,11- hexahydrotetracen-2-yl)oxy)tetrahydro-2H-pyran-4- yl)carbamoyloxy and
      **R⁶** is hydrogen; 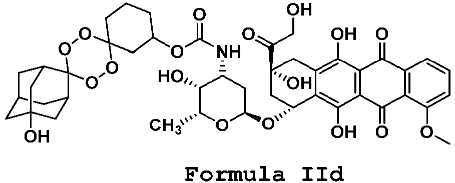 **wherein:**
         **R¹** is 5-hydroxy adamantyl
         **R²** is 5-hydroxy adamantyl
         **X** is -O-O-
         **R³** is -CH(R⁴)-CH(R⁵)-
         **R⁴** is hydrogen
         **R⁵** is (3-hydroxy-2-methyl-6-(((2S,4S)-4,5,12-trihydroxy-4-(2-hydroxyacetyl)-10-methoxy-6,11-dioxo-1,2,3,4,6,11-hexahydrotetracen-2-yl)oxy)tetrahydro-2H-pyran-4-yl) carbamoyloxy and
         **R⁶** is hydrogen; 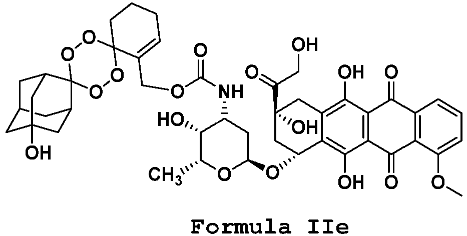 **wherein:**
            **R¹** is 5-hydroxy adamantyl
            **R²** is 5-hydroxy adamantyl
            **X** is -O-O-
            **R³** is -C(R⁴)=C(R⁵)-
            **R⁴** is (3-hydroxy-2-methyl-6-(((1R,3R)-3,5,12-trihydroxy-3-(2-hydroxyacetyl)-10-methoxy-6,11-dioxo-1,2,3,4,6,11-hexahydrotetracen-1-yl)oxy)tetrahydro-2H-pyran-4-yl)carbamoyloxy
            **R⁵** is hydrogen and
            **R⁶** is hydrogen.

Iron metabolism dysregulation occurs in diseases such as malaria and cancer. Therefore, the present invention also relates to biomarkers comprising said compounds of formula I.

Another aspect, the present invention relates to a process of synthesis of compounds of formula I, and respective intermediaries.

Further, the present invention also relates to a process for labelling, detection, and identification of tumours in a tissue sample.

The present invention is thus applicable to the medical and pharmacological areas, in particular the ones related to cancer detection and treatment, and malaria treatment.

The present invention discloses a drug delivery system with 2 different vias, based on a 1,2,4,5-tetraoxane scaffold that selectively react with Fe(II): while the first drug delivery system spontaneously releases the active pharmaceutical ingredient (API) after reaction with Fe(II), the second drug delivery system, in alternative to the first slightly modified, needs to react with Fe(II) and glutathione to release the active pharmaceutical ingredient (API).

### Background of the invention

Iron is a cofactor for enzymes involved in crucial biological processes such as mitochondrial respiration and cell cycle control. Its role depends on the redox cycle between ferric (Fe(III)) and ferrous iron (Fe(II)). However, it can also lead to reactive oxygen species (ROS) production by Fe(II) reaction with hydrogen peroxide. Despite being extremely important to several biological processes, iron can also be potentially harmful.

Iron homeostasis in cells maintains rigorous levels of iron that are adequate to assure its functions, while reducing its availability in the redox active ferrous form. An equilibrium between iron import, storage and export ensures iron homeostasis in cells. The mechanisms of iron metabolism are related by ferrous iron (redox active iron) that is named labile iron pool, mainly present in the cytosol.

Iron metabolism dysregulation occurs in diseases such as inflammation, infectious diseases, cancer, cardiovascular disease, and neurodegenerative diseases.

Endoperoxides such as artemisinin and its derivatives are used in the clinic for the treatment of malaria. Endoperoxides such as 1,2,4-trioxanes, 1,2,4-trioxolanes and 1,2,4,5-tetraoxanes are selectively activated by Fe(II)-rich parasite infected erythrocytes to form carbon-centred radicals. It appears that infected red blood cells contain elevated levels of Fe(II) when compared to those detected in healthy tissues.

Antimalarial drugs have harmful side effects and increasing resistance to the first line of treatment for malaria has been reported.

Several studies report a link between iron and cancer, showing that within cancer cells there are increased levels of the labile iron pool to support their biological needs. Moreover, many cancers modify iron import and export processes, leading to augmented levels of the labile iron pool, when compared with normal cells, which is a phenomenon named "iron addiction".

Currently, chemotherapeutic drugs are not selective towards their site of action. In consequence, they confer severe and harmful side effects.

Iron metabolism dysregulation represents a novel strategy for selective drug delivery by preparing two systems based on tetraoxanes. The drug delivery systems described in this invention can modify drug efficacy and drug resistance appearance since they are inactively administered and selectively activated towards the chosen target, in this case, the ferrous iron.

Document WO2015123595A1 describes compounds based on 1,2,4-trioxane or 1,2,4-trioxolane ring system for the treatment of parasitic and neoplastic diseases associated with conditions presenting higher level of ferrous iron.

However, document WO2015123595A1 is silent in what regards to delivery systems comprising compounds based on 1,2,4,5-tetraoxane. Moreover, as it is widely recognized in the literature, the 1,2,4-trioxolane ring system does not present good metabolic stability, which remains a major liability to their clinical development. The 1,2,4,5-tetraoxane ring system is thermodynamically and metabolically more stable than the 1,2,4-trioxolane ring system. Besides, these compounds are very toxic to the organism. Finally, the second drug delivery system disclosed is not amenable to contain the 1,2,4-trioxolane ring system due to limitations inherent to the preparation of the 1,2,4-trioxolane scaffold.

Document WO2020240266A1 discloses tetraoxanes compounds with biological activity against *Plasmodium* spp. such as Adamantane-2-spiro-3'-8'-hydroxymethyl-1',2',4'-trioxaspiro[4,5]decane-1,2-benzisothiazole-1,1-dioxide (LC129), Adamantane-2-spiro-3'-1',2',4',5'-tetraoxane-6'-spiro-1"-cyclohexanone (LC140), or Adamantane-2-spiro-3'-1',2',4',5'-tetraoxane-6'-spiro-1"-cyclohexane, (LC137) amongst other tetraoxanes compounds. However, the compounds herein disclosed are not able to selectively react with abnormally higher Fe(II) that occurs in iron metabolism dysregulation pathologies.

The present invention aims to overcome the problems of the prior art by providing new 1,2,4,5-tetraoxane compounds of formulae IIa, IIb, IIc, IId and IIe, and two drug delivery systems based in said compounds, which are selective to the targeted tissues, can contribute to the treatment of malaria and cancer, and further, can be successfully used as biomarkers.

### Description of the Figures

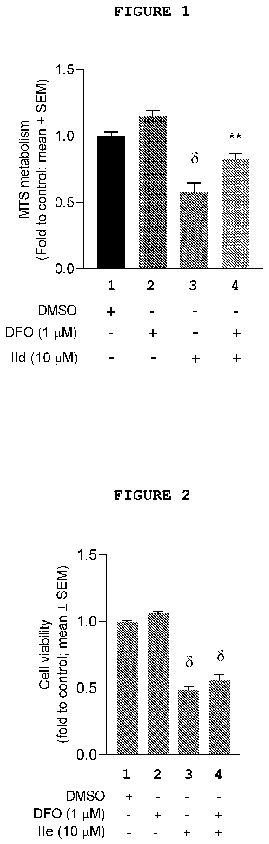

**Figure 1** shows the effect of cell death (Y axis) in colorectal cancer cells (HCT116) in the presence of compound **IId** alone, represented by column #3), of compound **IId** together with an iron chelator (deferoxamine mesylate - DFO), represented by column #4), of DFO alone, represented by column #2, and of dimethyl sulfoxide (DMSO) as a control compound, represented by column #1. On the Y axis is represented cell viability determined by the (3-(4,5-dimethylthiazol-2-yl)-5-(3-carboxymethoxyphenyl)-2-(4-sulfophenyl)-2H-tetrazolium (MTS) metabolism assay. Metabolically active cells (viable and alive) will metabolize the MTS into formazan and this molecule is quantified by absorbance. Higher amounts of formazan mean that the cells can survive when exposed to the compounds while lower amounts of formazan mean that the compounds are able to induce cell death.

From this figure it is possible to observe that compound **IId** induces cell death and that the co-treatment of compound **IId** with DFO promotes a lower induction of the cell death. Thus, cell death exerted by compound **IId** is iron-dependent. Data represent mean values ± standard error of the mean (SEM) of three independent experiments against to untreated control (DMSO). Ordinary One-Way ANOVA and Tukey's multiple comparisons test. Versus DMSO: δ *p <* 0.0001; Versus compound **IId** without DFO: ** *p* < 0.01.

**Figure 2** shows the effect of cell death (Y axis) in colorectal cancer cells (HCT116) in the presence of compound **IIe** alone, represented by column #3), of compound **IIe** together with an iron chelator (deferoxamine mesylate - DFO), represented by column #4), of DFO alone, represented by column #2, and of DMSO as a control compound, represented by column #1. On the Y axis is represented cell viability determined by the (3-(4,5-dimethylthiazol-2-yl)-5-(3-carboxymethoxyphenyl)-2-(4-sulfophenyl)-2H-tetrazolium (MTS) metabolism assay. Metabolically active cells (viable and alive) will metabolize the MTS into formazan and this molecule is quantified by absorbance. Higher amounts of formazan means that the cells can survive when exposed to the compounds while lower amounts of formazan means that the compounds are able to induce cell death.

From this figure it is possible to observe that compound **IIe** induces cell death. Also, the co-treatment of compound **IIe** with DFO promotes a small reduction in the induction of the cell death by compound **IIe.** Thus, cell death exerted by compound **IIe** can be iron-dependent because ferrous iron (Fe(II)) activates this compound in cells. Data represent mean values ± SEM of three independent experiments normalized to untreated control (DMSO). Ordinary One-Way ANOVA. Versus DMSO: δ p < 0.0001.

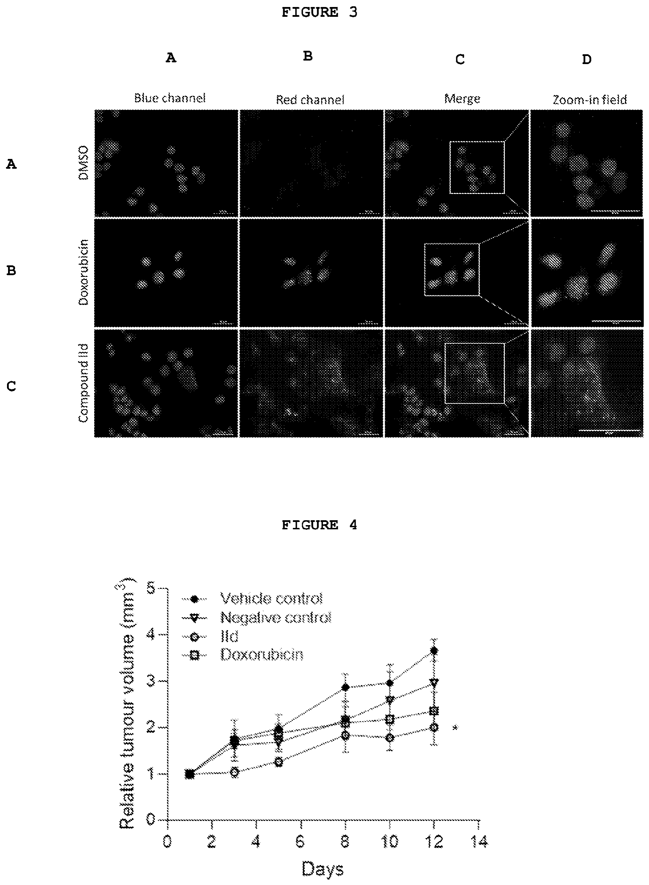

**Figure 3** presents images of HCT116 cells after 24 hours incubation with system control (DMSO), doxorubicin and compound **IId.** Fluorescence emission was detected using a 460 nm emission filter (blue channel) and a 560 nm emission filter (red channel). Hoechst dye 33258, is a fluorescent stain used to stain the cell nuclei and whose fluorescence emission can be detected by the blue channel. Doxorubicin has intrinsic fluorescence, and its emission wavelength is detected by the red channel. Merge is the overlap of fluorescence detected by the blue and red channels given by Hoechst dye 33258 and doxorubicin, respectively. AA represents system control (DMSO) and fluorescence emission in blue channel, AB is system control (DMSO) and fluorescence emission in red channel, AC represents the system control (DMSO) and merge of fluorescence emission from both channels (blue and red), AD represents the zoom-in field for the system control (DMSO) and merge of fluorescence emission from both channels (blue and red), BA is doxorubicin (1 µM) and fluorescence emission in blue channel, BB represents doxorubicin (1 µM) and fluorescence emission in red channel, BC represents doxorubicin (1 µM) and merge of fluorescence emission from both channels (blue and red), BD represents the zoom-in field for doxorubicin (1 µM) and merge of fluorescence emission from both channels (blue and red), CA is compound **IId** (1 µM) and fluorescence emission in blue channel, CB represents compound **IId** (1 µM) and fluorescence emission in red channel, CC is compound **IId** (1 µM) and merge of fluorescence emission from both channels (blue and red), CD represents the zoom-in field for compound **IId** (1 µM) and merge of fluorescence emission from both channels (blue and red). Objective: 63x. Scale bar = 50 µm.

From these images it is possible to observe that fluorescence in system control (DMSO) is only present in the blue channel meaning that only nuclei can be detected (images AA, AC and AD). It can be observed that doxorubicin accumulates in the nucleus through the co-localization of fluorescence of the blue and red channels (images BC and BD). In turn, compound **IId** presents fluorescence around the nuclei (images CC and CD). As compound **IId** has no intrinsic fluorescence, this means that compound **IId** is activated in the cytosol, where ferrous iron (Fe(II)) levels are elevated, and releases doxorubicin.

**Figure 4** shows the relative tumour volume (Y axis) for the four groups of mice injected with colorectal cancer cells (HT-29) during the days of treatment (X axis), for the mice treated with compound **IId,** represented by an unfilled circle, and doxorubicin, represented by an unfilled square. Mice without any treatment (negative control group) are represented by an unfilled and inverted triangle and the mice that received cremophor in phosphate buffered saline solution (vehicle control), which is the solvent to dissolve compound **IId** for administration, is represented by a filled circle. On the Y axis is represented the relative tumour volume in mm³ determined by the ratio between volumes at the indicated day and volume at the beginning of treatment for each group of mice.

From this figure it is possible to observe a reduction on the progression of the tumour mass for the mice treated with compound **IId** and doxorubicin when compared with the negative control group and the vehicle control. Moreover, compound **IId** was more efficient in reducing the tumour mass than doxorubicin. Mixed-effects analysis (two-way anova like) and Tukey's multiple comparisons test. At day 12, mice treated with compound **IId** presented a significant reduction in tumour volume versus vehicle control: * *p* < 0.05.

### Description of the invention

The present invention relates to novel drug delivery systems based on endoperoxide moieties such as 1,2,4,5-tetraoxanes, namely compounds of **formulae IIa, IIb, IIc, IId and IIe,** appropriately modified to release active pharmaceutical ingredients (API) in the presence of higher levels of Fe(II), in a subject.

### 1. Novel tetraoxane compounds

The tetraoxanes according to the present invention are compounds of the following formulae IIa, IIb, IIc, IId, IIe, including enantiomers and racemic mixtures thereof: 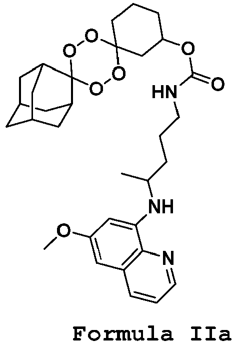 **Wherein:**
**R¹** is adamantyl
**R²** is adamantyl
**X** is -O-O-
**R³** is -CH(R⁴)-CH(R⁵
**R⁴** is hydrogen
**R⁵** is (4-((7-methoxyquinolin-5-yl)amino)pentyl) carbamoyloxy and
**R⁶** is hydrogen; 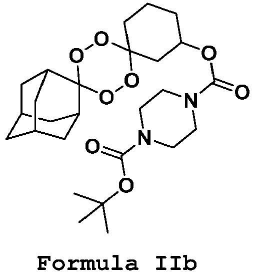 **Wherein:**
   **R¹** is adamantyl
   **R²** is adamantyl
   **X** is -O-O-
   **R³** is -CH(R⁴)-CH(R⁵)-
   **R⁴** is hydrogen
   **R⁵** is 1-(tert-butyl) piperazine-1,4-dicarboxylate and
   **R⁶** is hydrogen; 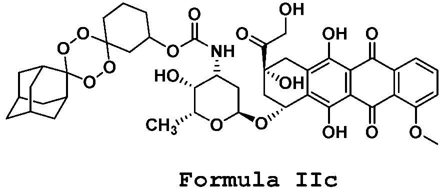 **wherein:**
      **R¹** is adamantyl
      **R²** is adamantyl
      **X** is -O-O-
      **R³** is -CH(R⁴)-CH(R⁵)-
      **R⁴** is hydrogen
      **R⁵** is (3-hydroxy-2-methyl-6-((2S,4S)-4,5,12-trihydroxy-4-(2- hydroxyacetyl)-10-methoxy-6,11-dioxo-1,2,3,4,6,11- hexahydrotetracen-2-yl)oxy)tetrahydro-2H-pyran-4- yl)carbamoyloxy and
      **R⁶** is hydrogen; 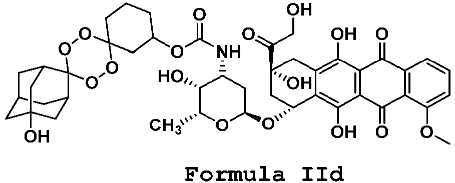 **wherein:**
         **R¹** is 5-hydroxy adamantyl
         **R²** is 5-hydroxy adamantyl
         **X** is -O-O-
         **R³** is -CH(R⁴)-CH(R⁵)-
         **R⁴** is hydrogen
         **R⁵** is (3-hydroxy-2-methyl-6-(((2S,4S)-4,5,12-trihydroxy-4-(2- hydroxyacetyl)-10-methoxy-6,11-dioxo-1,2,3,4,6,11- hexahydrotetracen-2-yl)oxy)tetrahydro-2H-pyran-4-yl) carbamoyloxy and
         **R⁶** is hydrogen; 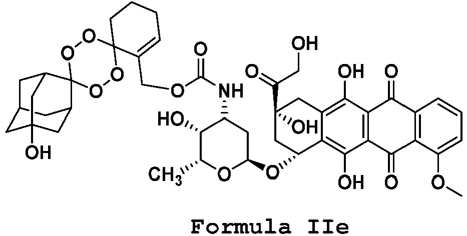 **wherein:**
            **R¹** is 5-hydroxy adamantyl
            **R²** is 5-hydroxy adamantyl
            **X** is -O-O-
            **R³** is -C(R⁴)=C(R⁵)-
            **R⁴** is (3-hydroxy-2-methyl-6-(((1R,3R)-3,5,12-trihydroxy-3-(2-hydroxyacetyl)-10-methoxy-6,11-dioxo-1,2,3,4,6,11- hexahydrotetracen-1-yl)oxy)tetrahydro-2H-pyran-4- yl)carbamoyloxy
            **R⁵** is hydrogen and
            **R⁶** is hydrogen,
which are appropriately modified to release active pharmaceutical ingredients (API) in the presence of higher levels of Fe(II), in a subject.

### 1.1. Characteristics and properties of the compounds of the invention for malaria treatment (formula IIa and IIb)

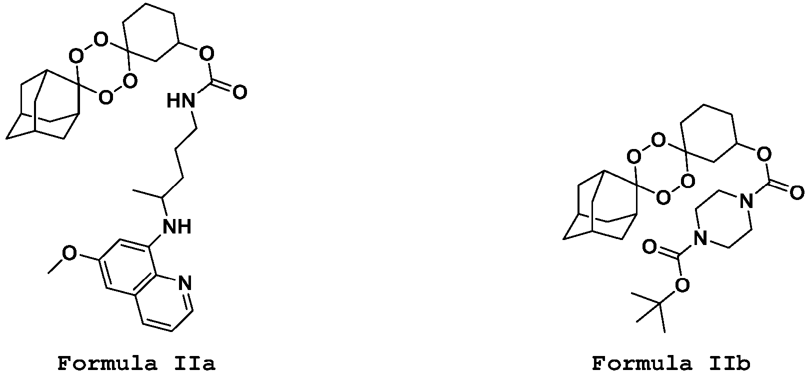

Compound **IIa** and **IIb** are coupled with drugs for malaria treatment defining a drug delivery system **IIa** and **IIb.** In a preferred embodiment, compound **IIa** is coupled with primaquine, an antimalarial drug used in the clinic. In another embodiment, compound **IIb** is coupled with boc-piperazine. These compounds exhibit a half maximal inhibitory concentration (IC₅₀) in the nanomolar range against chloroquine-sensitive 3D7 strain of *Plasmodium falciparum.* Therefore, these compounds can be applied in malaria treatment. Compounds **IIa** and **IIb** also include respective racemic mixture or respective enantiomers.

### 1.2. Characteristics and properties of the compounds of the invention for cancer treatment (formulae IIc, IId and IIe)

Further compounds are in the scope of the present invention, such as compound of formula **IIc, IId** and **IIe.**

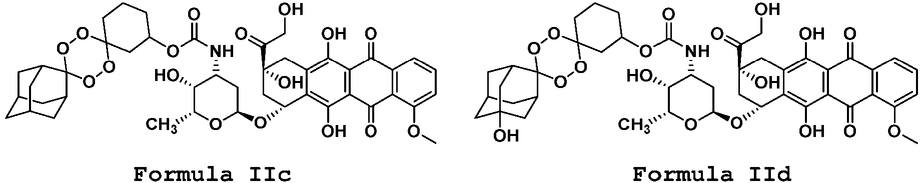

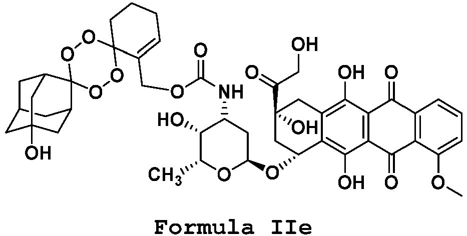

Compounds **IIc, IId** and **IIe** are coupled with an anticancer drug typically used in the clinic thus defining drug delivery systems **IIc, IId** and **IIe.** In a preferred embodiment, the compounds of the invention are coupled with doxorubicin. These compounds are able to induce cell death *in vitro,* in the micromolar range, for at least four types of cancer with iron metabolism instability, wherein the specific cell lines are identified between brackets: glioblastoma (U251), breast adenocarcinoma (MCF7), prostate adenocarcinoma (PC3) and colorectal carcinoma (HCT116). Thus, these compounds can be applied in cancer treatment. Compounds **IIc, IId** and **IIe** also include respective racemic mixture or respective enantiomers.

### 1.3. Characteristics and properties of the compounds of the invention for cancer diagnostic (formulae IIc, IId and IIe)

Besides its ability to induce cell death in cancer cells, some other compounds having intrinsic fluorescence, such as doxorubicin are useful to be used as tumour markers, when coupled with compounds of the invention. This fluorescence is lost when said compounds are linked to drug compounds **IIc, IId** and **IIe.** When these fluorescent compounds are activated in cancer cells, upon reaction with ferrous iron, said fluorescent compound is released from **IIc, IId** and **IIe,** thus, recovering its fluorescence properties, which is detected by fluorescence microscopy. Therefore, these compounds can also be employed as drug delivery systems for tumour markers. In a preferred embodiment doxorubicin is linked to compounds **IIc, IId** and **IIe** to be used as tumour markers.

### 2. Process of preparation of compounds of formula I

In an embodiment of the present invention, the process for preparation of compounds of **formula I** (see Example 1) and of compounds of formulae **IIa, IIb, IIc** and **IId** (see Example 2), as described in the previous section is represented by scheme 1 below: 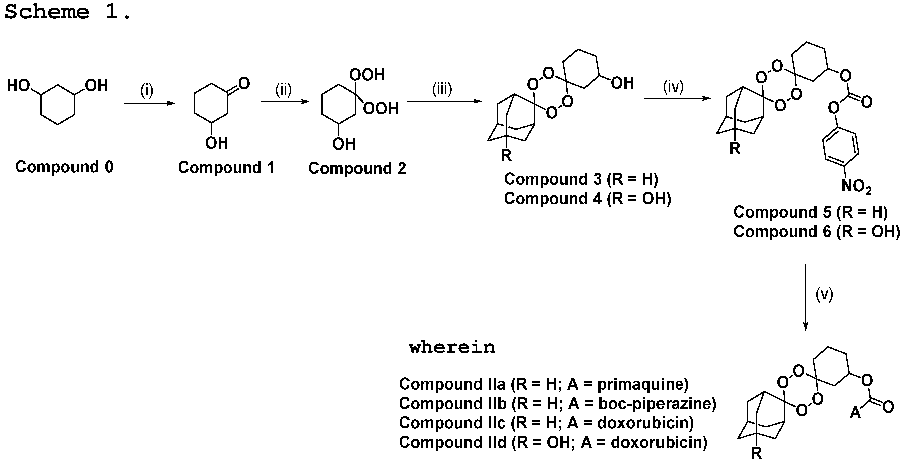 with the following steps:
(i) Sodium dichromate dihydrate, water, sulfuric acid;
(ii) Rhenium (VII) oxide, hydrogen peroxide 50%, acetonitrile;
(iii) 2-Adamantanone or 5-hydroxy-2-adamantanone, rhenium (VII) oxide, dichloromethane:acetonitrile (1:1), inert atmosphere;
(iv) 4-Nitrophenyl chloroformate, *N,N*-diisopropylethylamine, 4-dimethylaminopyridine, dichloromethane;
(v) API, 1-hydroxybenzotriazole hydrate, triethylamine, *N,N-*dimethylformamide.

In **step i)** there is the oxidation of 1,3-cyclohexanediol (Compound **0**) generating rac-3-hydroxycyclohexanone **(Compound 1).**

In **step ii),** Compound **1** reacts with hydrogen peroxide in the presence of rhenium(VII) oxide to produce the dihydroperoxide **(Compound 2).**

In **step iii)** the dihydroperoxide **(Compound 2)** reacts with a carbonyl reagent, employing again a catalytic amount of rhenium(VII) oxide. 2-Adamantanone **(Compound 3)** and 5-hydroxy-2-adamanatone **(Compound 4)** can be used as carbonyl for the formation of the 1,2,4,5-tetraoxane scaffold of the first drug delivery system. The alcohol group on position 3" of the cyclohexane ring generates unsymmetrical tetraoxanes **(compounds 3 and 4)** with a stereogenic center, obtaining 2 enantiomers. Thus, this step may also employ a racemic mixture of tetraoxanes to be applied as a drug delivery system instead of performing a stereocontrolled synthesis of the 1,2,4,5-tetraoxane system.

Next, in **step iv)** compounds **(3)** and **(4)** were activated with 4-nitrophenyl chloroformate together with excess amine bases (*N,N-*diisopropylethylamine and 4-dimethylaminopyridine) to generate compounds **(5)** and **(6),** respectively.

Finally, compound **(5)** was coupled with primaquine.diphosphate **(IIa)** and boc-piperazine **(IIb)** with 55% and 33% yields, respectively. Compound **(5)** and **(6)** were coupled with doxorubicin to obtain the carbamates with 60% **(IIc)** and 75% **(IId),** producing the correspondent drug delivery system.

Compounds **1 to 6** with the above-described formulae, and enantiomers of compounds **3** to **6** are included in the present invention as intermediaries in the process of production of compounds **IIa, IIb, IIc and IId** and respective drug delivery systems.

Finally, considering that the intermediary compounds **3 to 6** are used in the process of preparing compounds **IIa, IIb, IIc and IId,** this results that said compounds and respective drug delivery systems include a racemic mixture or respective enantiomers. Accordingly, the present invention also relates to racemic mixture or respective enantiomers of compounds **IIa, IIb, IIc and IId** and correspondent drug delivery systems.

In another embodiment of the invention, the process for preparation of compounds of **formula I (see** Example 1), and of compounds of formulae **IIe** (see Example 2), is described in scheme 2 below: 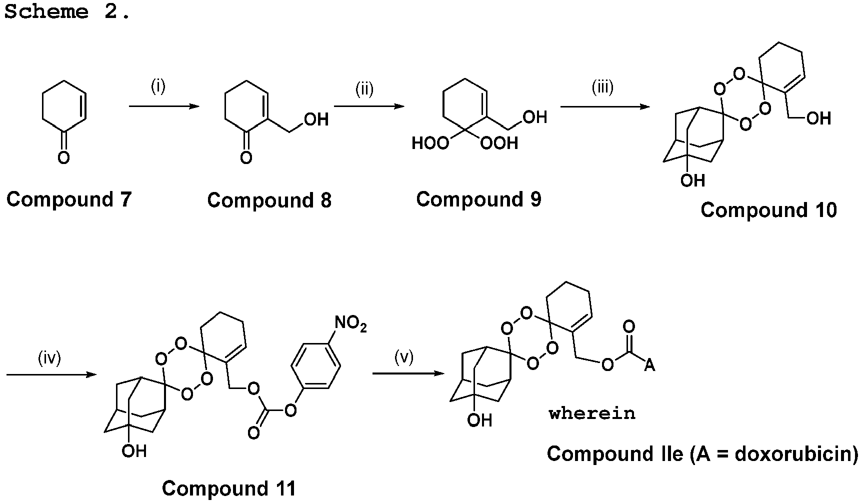 with the following steps:
(i) Formaldehyde, N-methylpyrrolidine, barium hydroxide octahydrate, water:methanol (5:1);
(ii) Rhenium (VII) oxide, hydrogen peroxide 50%, acetonitrile;
(iii) 5-Hydroxy-2-adamantanone, rhenium (VII) oxide, dichloromethane:acetonitrile (1:1), inert atmosphere;
(iv) 4-Nitrophenyl chloroformate, N,N-diisopropylethylamine, 4-dimethylaminopyridine, dichloromethane;
(v) API, 1-hydroxybenzotriazole hydrate, triethylamine, N,N-dimethylformamide.

In **step i)** the hydroxymethyl group was installed on the position 2 of 2-cyclohexen-1-one **(compound 7)** by Morita-Baylis-Hillman chemistry, generating compound **8.**

In **step ii),** Compound **8** reacts with hydrogen peroxide in the presence of rhenium(VII) oxide to produce the dihydroperoxide **(Compound 9**).

In **step iii)** the dihydroperoxide **(Compound 9)** reacts with a carbonyl reagent, employing again a catalytic amount of rhenium(VII) oxide. 5-hydroxy-2-adamanatone **(Compound 10)** can be used as carbonyl for the formation of the 1,2,4,5-tetraoxane scaffold of the second drug delivery system. The alcohol group on position 5 of the adamantyl group generates an unsymmetrical tetraoxane **(compound 10)** with a stereogenic center, obtaining 2 enantiomers. This step may also employ a racemic mixture of tetraoxanes to be applied as a drug delivery system instead of performing a stereocontrolled synthesis of this drug delivery system.

Next, in **step iv)** compound **(10)** was activated with 4-nitrophenyl chloroformate together with excess amine bases (*N,N-*diisopropylethylamine and 4-dimethylaminopyridine) to generate compound **(11).**

Finally, compound **(11)** was coupled with doxorubicin **(IIe)** with 73% yield.

Compounds **8 to 11** with the above-described formulae, and enantiomers of compounds **10** and **11** are included in the present invention as intermediaries in the process of production of compounds of **formula I** and of **formula IIe.**

Finally, considering that the intermediary compounds **10 and 11** are used in the process of preparing the above-mentioned compounds, this results that said compounds include a racemic mixture or respective enantiomers. Accordingly, the present invention also relates to racemic mixture or respective enantiomers of **compounds I** and **IIe.**

### 3. Mechanism of the first drug delivery system according to the invention

The first drug delivery system contains the 1,2,4,5-tetraoxane scaffold that reacts with Fe(II) and forms a cyclohexanone intermediate (Scheme 3). This intermediate spontaneously releases the API by retro-Michael reaction (β-elimination).

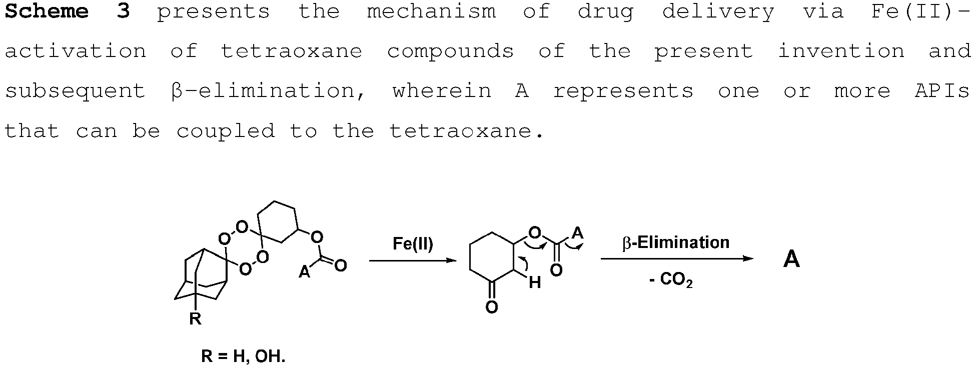

The reaction of the tetraoxane present in compounds **IIa, IIb, IIc** and **IId** with a ferrous iron source will produce a cyclohexanone derivative, which spontaneously releases the API by β-elimination (Scheme 3).

In the scope of the present invention, there are several suitable APIs that can be used alone or in combination for different purposes of treatment and/or diagnosis being some of them mentioned below.

In this scheme, compounds **IIa** and **IIb** can be coupled with one or more APIs against the **malaria** parasite such as primaquine and boc-piperazine. Other compounds suitable to couple with this system for malaria are the following and their derivatives: atovaquone, quinine sulphate, sulphonamides (sulphadoxine and sulphamethoxypyridazine) and falcipain inhibitors (dipeptidyl vinyl sulfones and peptidomimetic pyrimidine nitriles).

Compounds **IIc** and **IId** can be coupled preferably with doxorubicin, an API against cancer. Other compounds suitable to couple with this system for cancer either alone or in combination are the following and their derivatives: alectinib, afatinib, abiraterone acetate, azacitidine, axitinib, bendamustine, brigatinib, bosutinib, bicalutamide, calicheamicin, capecitabine, carfilzomib, combretastatin, ceritinib, cladribine, clofarabine, cobimetinib, crizotinib, cryptophycin, cytarabine, dabrafenib, dasatinib, daunorubicin, decitabine, duocarmycin, enasidenib, epirubicin, eribulin mesylate, erlotinib, everolimus, etoposide, exatecan, fludarabine, fulvestrant, gemcitabine, hemiasterlin, histone deacetylase inhibitors (abexinostat, belinostat, entinostat, givinostat, mocetinostat, oxamflatin, panobinostat, pyroxamide, quisinostat, vorinostat, trichostatin A, tacedinaline, tucidinostat, tubastatin A), hydroxyurea, ibrutinib, idelalisib, imatinib, irinotecan, ixabepilone, lapatinib, lenalidomide, maytansine, mitomycin C, monomethyl auristatins (MMA-E, MMA-F, dolastatin 10 and dolastatinol), methotrexate, neratinib, nilotinib, niraparib, olaparib, octreotide, osimertinib, paclitaxel, palbociclib, pazopanib, pyrrolobenzodiazepine, pemetrexed, pomalidomide, ponatinib, ribociclib, rucaparib, sorafenib, tamoxifen, triazenes, trifluridine, topotecan, tubulysin, vandetanib.

Other compounds suitable to couple with this system as tumour markers includes fluorophores 4,4-difluoro-4-bora-3a,4a-diaza-s-indacene (BODIPY), cyanines, dicyanomethines, doxorubicin, fluorescein, nitrobenzofurazan, rhodamine and derivatives thereof.

### 3.1. Mechanistic proof-of-concept of the first drug delivery system

The activation of the 1,2,4,5-tetraoxane scaffold after reaction with Fe(II) causes the production of carbon-centred radicals. This reaction mechanism involves Fe(II) coordination with two possible oxygens of the tetraoxane ring. The first drug delivery system contain a tetraoxane scaffold that, in the presence of Fe(II), is expected to produce carbon-centred radicals and a cyclohexanone derivative. This derivative will spontaneously release the API by β-elimination. To confirm the proposed mechanism of drug delivery, compound **IIc** was exposed to an inorganic salt of Fe(II) and 2,2,6,6-tetramethylpiperidine-1-oxyl radical (TEMPO), as described in O'Neill et. al, J. Med. Chem. 2011, 54, 6443. TEMPO was used in this experiment to validate the formation of carbon-centred radicals by the drug delivery system. TEMPO reacts with the radicals generated in this reaction and allows their detection by ultra-high performance liquid chromatography-mass spectrometry (UHPLC-MS).

Fe(II)-triggered tetraoxane activation and fragmentation of compound **IIc** detected TEMPO adducts by UHPLC-MS consistent with the formation of carbon-centred radicals (see Example 3). Moreover, the cyclohexanone derivative and doxorubicin were also identified by UHPLC-MS (see Example 3), which confirms the API release by the proposed mechanism of drug delivery.

### 4. Mechanism of the second drug delivery system disclosed in this invention

The alternative second drug delivery system, which is a particular embodiment of the first system described above, also contains the 1,2,4,5-tetraoxane scaffold, which after reaction with Fe(II), produces a cyclohex-2-enone derivative. This derivative does not release the API spontaneously. This derivative possesses an alkene group that after reaction with glutathione (Michael addition) allows the release of the API by elimination (Scheme 4). Considering that API release only occurs after reaction with Fe(II) and glutathione, which is elevated in many types of cancer, this can lead to improved selectivity when compared with the first drug delivery system.

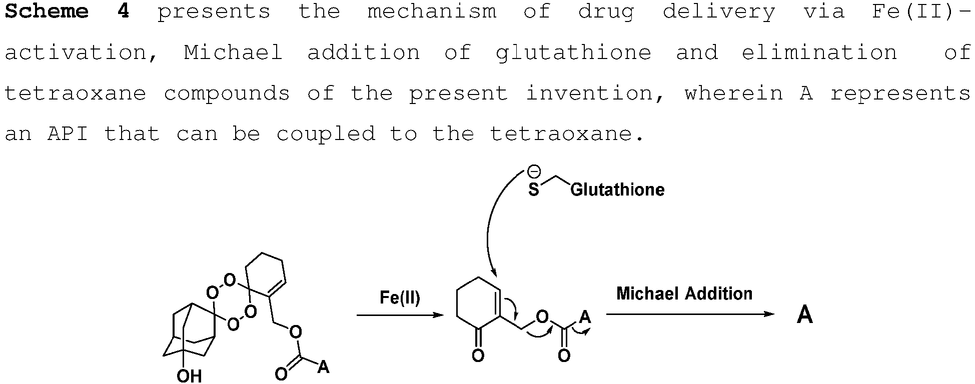

The reaction of the tetraoxane present in compound **IIe** with a ferrous iron source will produce a cyclohex-2-enone derivative, which releases the API by elimination after reacting with glutathione (Scheme 4).

In the scope of the present invention, there are several suitable APIs that can be used alone or in combination for different purposes of treatment and/or diagnosis being some of them mentioned below.

In this scheme, compound **IIe** is preferably coupled with doxorubicin, an API against cancer. Other compounds suitable to couple with this system for cancer alone or in combination are the following and their derivatives: alectinib, afatinib, abiraterone acetate, azacitidine, axitinib, bendamustine, brigatinib, bosutinib, bicalutamide, calicheamicin, capecitabine, carfilzomib, combretastatin, ceritinib, cladribine, clofarabine, cobimetinib, crizotinib, cryptophycin, cytarabine, dabrafenib, dasatinib, daunorubicin, decitabine, duocarmycin, enasidenib, epirubicin, eribulin mesylate, erlotinib, everolimus, etoposide, exatecan, fludarabine, fulvestrant, gemcitabine, hemiasterlin, histone deacetylase inhibitors (abexinostat, belinostat, entinostat, givinostat, mocetinostat, oxamflatin, panobinostat, pyroxamide, quisinostat, vorinostat, trichostatin A, tacedinaline, tucidinostat, tubastatin A), hydroxyurea, ibrutinib, idelalisib, imatinib, irinotecan, ixabepilone, lapatinib, lenalidomide, maytansine, mitomycin C, monomethyl auristatins (MMA-E, MMA-F, dolastatin 10 and dolastatinol), methotrexate, neratinib, nilotinib, niraparib, olaparib, octreotide, osimertinib, paclitaxel, palbociclib, pazopanib, pyrrolobenzodiazepine, pemetrexed, pomalidomide, ponatinib, ribociclib, rucaparib, sorafenib, tamoxifen, triazenes, trifluridine, topotecan, tubulysin, vandetanib.

Other compounds suitable to couple with this system as tumour markers includes fluorophores 4,4-difluoro-4-bora-3a,4a-diaza-s-indacene (BODIPY), cyanines, dicyanomethines, doxorubicin, fluorescein, nitrobenzofurazan, rhodamine and derivatives thereof.

### 4.1. Mechanistic proof-of-concept of the second drug delivery system

As described for the first drug delivery system, the tetraoxane reaction with a ferrous iron source of the second drug delivery system also produces carbon-centred radicals, by Fe(II) coordination with two oxygens of the tetraoxane scaffold. The second drug delivery system generate a cyclohex-2-enone derivative upon activation by Fe(II), which then undergo a Michael addition by cellular glutathione and subsequent departure of the API by elimination. To confirm the first step of the proposed mechanism of drug delivery, compound **IIe** was exposed to an inorganic salt of Fe(II) and TEMPO, as described in O'Neill et. al, J. Med. Chem. 2011, 54, 6443. TEMPO was used in this experiment to validate the formation of carbon-centred radicals by the drug delivery system. TEMPO reacts with the radicals generated in this reaction and allows their detection by UHPLC-MS. Moreover, this experiment was performed to confirm tetraoxane fragmentation of compound **IIe** producing the cyclohex-2-enone derivative. Finally, doxorubicin is not released under these conditions because of the absence of glutathione or similar agents, therefore, the Michael addition to the cyclohex-2-enone derivative cannot occur.

Fe(II)-triggered tetraoxane activation and fragmentation of compound **IIe** detected TEMPO adducts by UHPLC-MS (see Example 4), for the two possibilities of coordination with Fe(II) and consist with the formation of carbon-centred radicals. Moreover, the cyclohex-2-enone derivative was also identified by UHPLC-MS (see Example 4), in accordance with the proposed mechanism. Finally, in this experiment doxorubicin was not detected.

This drug delivery system produces a cyclohex-2-enone derivative that after activation by Fe(II) can undergo a Michael addition by cellular glutathione and, subsequent release of the API. To confirm this mechanism of drug delivery, compound **IIe** reacted with a ferrous iron source and N-acetylcysteine (NAC), to simulate the Michael addition of glutathione, which relies on a thiol group (-SH). In this example, to assure that this reaction occurs, a pH over 8 is necessary for the thiol group to be deprotonated (-S⁻).

Fe(II)-triggered tetraoxane activation and fragmentation of compound **IIe** detected a carbon-centred radical by UHPLC-MS (see Example 4). Additionally, the cyclohex-2-enone derivative was also detected (see Example 4), in accordance with the first step of activation of this drug delivery system. Finally, release of doxorubicin was detected (see Example 4), validating the mechanism of action for this drug delivery system because Michael addition of NAC to the cyclohex-2-enone derivative occurred.

### 5. Biological activity assessment

The biological activity of the compounds and correspondent drug delivery systems of the invention can be accessed by measurement of different parameters, in line with the known processes by the skilled person in the art.

### 5.1. Evaluation of the antimalarial effect

Antimalarial activity of compounds **IIa** and **IIb** can be accessed by exposing a *P. falciparum* strain (see Example 5), such as strain 3D7, which is chloroquine and mefloquine sensitive, to said compounds, and by using the SYBR Green I assay as described in Machado et. al, Ann. Clin. Med. Microbio. 2016, 2, 1010.

Asynchronous parasites are cultivated for 72 hours in the presence of each compound. Fluorescence intensity was measured with a multi-mode microplate reader (Triad, Dynex Technologies). Compounds **IIa** and **IIb** were active against the malaria parasite (see Example 5, Table 1).

### 5.2. Evaluation of the antitumoral effect

Typically, the antitumoral effect against cancer cells growth is accessed by measuring the half maximal inhibitory concentration (IC₅₀) in a cell culture exposed to the compounds to be evaluated, according to the described in Florindo et. al, Dalton Trans 2016, 45, 11926.

The IC₅₀ values can be calculated by dose-response curves, such as by using GraphPad Prism 8.0.2. software followed by statistical analysis.

For this purpose, tumour cell lines are exposed to a range of concentrations of suitable compounds **IIc, IId** and **IIe** and correspondent drug delivery systems being cells metabolic activity evaluated after 72 hours by CellTiter 96^{®} Aqueous Non-Radioactive Cell Proliferation Assay (3-(4,5-dimethylthiazol-2-yl)-5-(3-carboxymethoxyphenyl)-2-(4-sulfophenyl)-2H-tetrazolium - MTS; Promega, Madison, WI, USA) and absorbance signal recorded using a GloMax^{®}-MultiDetection System (Promega). Compounds **IIc, IId** and **IIe** were able to induce cell death in all the tested tumour cell lines (see Example 6, Table 2). Moreover, compounds **IIc, IId** and **IIe** were less potent than doxorubicin, which is the API against cancer coupled in these compounds.

### 5.3. Evaluation of the effect of compounds in toxicity

Compounds **IIc, IId** and **IIe** and doxorubicin were tested on a non-tumorigenic cell line (AML12) to assess the toxicity of the compounds described in this invention. The toxicity can be measured by calculating the IC₅₀ of the compounds in this non-tumoral cell line. The conditions of the assay and the software can be the same as described previously for compounds **IIc, IId** and **IIe** against the four tumour cell lines. Compounds **IIc, IId** and **IIe** were able to ameliorate the toxicity associated with doxorubicin (see Example 7).

### 5.4. Evaluation of biological activity dependent on iron

Compound **IId** and **IIe** were incubated alone and together with the iron chelator desferoxamine mesylate (DFO) in colorectal cancer cells (HCT116), as described in Mariani et. al, Chem. Commun. 2016, 52, 1358 (see Example 8). The cellular metabolic activity can be determined again with the MTS assay and absorbance signal detection, as described above for the evaluation of the antitumoral effect. The IC₅₀ calculations and statistical analysis can also be performed by GraphPad Prism 8.0.2.

The induction of cell death by compound **IId** relies on iron because the cytotoxicity of this compound was reduced in the presence of the iron chelator DFO (Figure 1). Therefore, the presence of Fe(II) is essential for the activity of the first drug delivery system in cells.

The second drug delivery system contains an alkene group as an additional element of selectivity when compared with the first drug delivery system. Compound **IIe** is activated in cells by iron, but the major contributor of the cytotoxicity is related with the Michael addition by cellular glutathione. The induction of cell death by compound **IIe** showed a small reduction in the cytotoxicity of this compound in the presence of the iron chelator DFO (Figure 2). Therefore, in this example the presence of Fe(II) activates the second drug delivery system in cells. This activation generates the cyclohex-2-enone derivative, which contains an alkene group, that after reaction with cellular glutathione allows the release of the API, which is further described in example 4.

### 5.5. Compounds of formula II as tumour markers

Evaluation of drug delivery systems of the invention **IIc, IId** and **IIe** as tumour markers was performed using fluorescence microscopy to detect their cellular localization. Compound **IId** and doxorubicin were incubated in colorectal cancer cells (HCT116) and after 24 hours of incubation images were acquired with a Zeiss Axio Scope.A1 microscope coupled with an AxioCam HR R3 camera and images analysed with Zen Software 2012 Blue Edition. Compound **IId** was present in a different cellular localization when compared with doxorubicin (see Example 9 and Figure 3).

### 5.6. In vivo validation for the first drug delivery system

Compound **IId** and doxorubicin were tested for colorectal cancer using HT-29 cells in a xenograft murine model. During the treatment, the tumour size was analysed and used to determine the tumour volume and the relative tumour volume. Twenty immunocompromised Foxn1^{nu/nu} mice were divided into four groups: negative control, vehicle control, compound **IId** and doxorubicin. The negative control group is composed of five mice that did not receive treatment. The vehicle control is composed of five mice that received the solvent used to dissolve compound **IId.** Finally, five mice were treated with compound **IId** and five mice were treated with doxorubicin. Compound **IId** and doxorubicin were more efficient in reducing the relative tumour mass while comparing with the control groups (Figure 4). The reduction of the relative tumour mass was higher for mice treated with compound **IId** when compared with mice treated with doxorubicin (Figure 4).

### EXAMPLES

### Example 1. Synthesis of 1,2,4,5-tetraoxane compounds of formula I

Cyclocondensation of dihydroperoxide **(2)** in the presence of a carbonyl (2-adamantanone or 5-hydroxy-2-adamantanone) produced 1,2,4,5-tetraoxane scaffolds **(3)** and **(4).** Alcohol activation in position 3 with 4-nitrophenyl chloroformate generated compounds **(5)** and **(6).** Finally, API coupling with compounds **(5)** and **(6)** produced the four compounds **(IIa** to **IId)** described in this invention for the first drug delivery system.

Cyclocondensation of dihydroperoxide **(9)** in the presence of a carbonyl reagent, in this case 5-hydroxy-2-adamantanone, produced the 1,2,4,5-tetraoxane scaffold **(10).** Alcohol activation of compound **(10)** with 4-nitrophenyl chloroformate generated compound **(11).** Finally, API coupling with compound **(11)** produced the compound **IIe** described in this invention for the second drug delivery system.

For this purpose, several intermediary compounds **(1** to **6** and **8** to **11)** were produced, according to the described below.

### 1.1. Synthesis of compound (1): rac-3-hydroxycyclohexanone

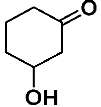

A sodium dichromate solution (3.01 g, 10.3 mmol) in concentrated sulfuric acid/water (1.7 mL/12 mL) was added to a 1,3-cyclohexanediol (3.45 g, 29.7 mmol) solution in diethyl ether (12 mL) dropwise. After complete addition, the reaction was left to stir at 25°C for 3.5 hours. The reaction mixture was extracted with ethyl acetate (3x30 mL), the organic fractions combined, dried over anhydrous sodium sulphate, filtered, and evaporated. Purification by flash chromatography using Hexane:Ethyl acetate(2:8) as eluent isolated compound **1** as a colourless oil (1.35 g, 11.8 mmol) and 1,3-cyclohexanediol (1.26 g, 10.8 mmol).

**Yield:** 63%; **¹H NMR (300 MHz, CDCl₃)** : δ (ppm) 4.18-4.10 (m, 1H), 2.93 (br s, 1H), 2.60 (dd, J = 14.1, 4.2 Hz, 1H), 2.37 (dd, J = 13.8, 7.5 Hz, 1H), 2.27 (t, J = 6.6 Hz, 2H), 2.09-1.88 (m, 2H), 1.80-1.55 (m, 2H); **¹³C NMR (75 MHz, CDCl₃):** δ (ppm) 210.4, 69.8, 50.5, 41.0, 32.8, 20.8; **MS (ES⁺):** [M+H]⁺ 114.9 (12).

### 1.2. Synthesis of compound (2): 3,3-dihydroperoxycyclohexan-1-ol

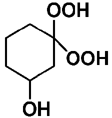

A 25 mL round-bottom flask was charged with rac-3-hydroxycyclohexanone (0.219 g, 1.92 mmol) and rhenium(VII) oxide (0.044 g, 0.092 mmol) in acetonitrile (10,5 mL). Then, hydrogen peroxide 50% (0.40 mL, 7.0 mmol) was added at room temperature and left to stir for 40 minutes. The mixture was filtered under vacuum through a silica plug and washed with ethyl acetate. The solution was concentrated, and compound **2** was isolated as a colourless oil (0.313 g, 1.91 mmol).
**Yield:** 100%; **¹H NMR (300 MHz, (CD₃)₂CO):** δ (ppm) δ 9.99 (s, 1H), 9.67 (s, 1H), 3.91-3.63 (m, 2H), 2.46-2.36 (m, 1H), 2.13-2.07 (m, 1H), 1.91-1.82 (m, 1H), 1.69-1.59 (m, 1H), 1.53-1.14 (m, 4H); **¹³C NMR (75 MHz, (CD₃)₂CO):** δ (ppm) 110.7, 67.5, 39.6, 39.4, 35.6, 20.4;

### 1.3. Synthesis of compound (3): dispiro[adamantane-2,3'-[1,2,4,5]tetraoxane-6',1"-cyclohexan]-3"-ol

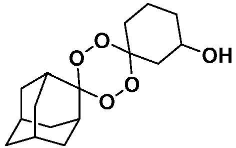

A 25 mL round-bottom flask was charged with 2-adamantanone (0.433 g, 2.88 mmol), rhenium(VII) oxide (0.020 g, 0.041 mmol) in dichloromethane (5.7 mL) under inert atmosphere. Then, 3,3-dihydroperoxycyclohexan-1-ol (0.313 g, 1.91 mmol) in acetonitrile (5.7 mL) was added at room temperature and left to stir for 45 minutes. The mixture was filtered under vacuum through a silica plug and washed with ethyl acetate. The solution was concentrated and purification by flash chromatography in Hexane:Ethyl acetate (7:3) isolated compound 3 as a white solid (0.315 g, 1.06 mmol).

**Yield:** 56%; **¹H NMR (300 MHz, CDCl₃):** δ (ppm) 4.52-4.45 (m, 1H), 3.11-3.03 (m, 1H), 2.16-1.67 (m, 21H); **¹³C NMR (75 MHz, CDCl₃):** δ (ppm) 110.7, 73.2, 41.3, 37.0, 35.8, 33.8, 33.2, 31.0, 27.1, 25.9; **MS (ES⁺):** [M+H]⁺ 297.2 (10); [M-Tetraoxane cleavage]⁺ 167.0 (100).

### 1.4. Synthesis of compound (4): dispiro[adamantane-2,3'-[1,2,4,5]tetraoxane-6',1"-cyclohexane]-3",5-diol

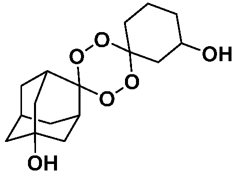

For the synthesis of compound **4** the same method was used with 3,3-dihydroperoxycyclohexan-1-ol (0.141 g, 0.861 mmol) as starting material; however, 2-adamantanone was replaced by 5-hydroxy-2-adamantanone (0.216 g, 1.30 mmol). Purification by flash chromatography in Hexane:Ethyl acetate (2:8) isolated compound **4** as a white solid (0.145 g, 0.464 mmol).

**Yield:** 54%; **¹H NMR (300 MHz, CDCl₃):** δ (ppm) 4.63-4.59 (m, 1H), 3.18-3.12 (m, 1H), 2.59-2.39 (m, 2H), 2.16-1.50 (m, 18H); **¹³C NMR (100 MHz, CDCl₃):** δ (ppm) 108.6, 74.6, 66.7, 43.7, 42.1, 41.5, 38.8, 34.7, 30.4, 29.9; **MS (ES⁺):** [M-Tetraoxane cleavage]⁺ 183.0 (100). **Elemental Analysis:** Found: C, 61.46; H, 7.60. C₁₆H₂₄O₆ requires C, 61.52; H, 7.74.

### 1.5. Synthesis of compound (5): dispiro[adamantane-2,3'-[1,2,4,5]tetraoxane-6',1"-cyclohexan]-3''-yl (4-nitrophenyl) carbonate

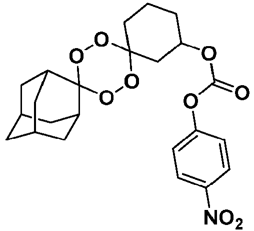

Compound **3** (0.120 g, 0.404 mmol) was dissolved in dichloromethane (1.6 mL) and, afterwards, N,N-diisopropylethylamine (0.21 mL, 1.21 mmol) and 4-dimethylaminopyridine (0.0520 g, 0.421 mmol) were added to the solution. At 0°C, 4-nitrophenyl chloroformate (0.168 g, 0.814 mmol) was added and the reaction was left to stir under inert atmosphere for 15 minutes, 0°C to room temperature for 10 minutes and at room temperature for 20 minutes. The reaction mixture was diluted in diethyl ether (30 mL), washed with 5% potassium bisulphate (10 mL) and sodium bicarbonate saturated solution (5x10 mL). The organic phase was washed with brine (1x25 mL), dried with anhydrous sodium sulphate, and afterward was filtered and concentrated. Purification by flash chromatography in Hexane:Ethyl acetate (9:1) isolated compound 5 as a white solid (0.0416 g, 0.090 mmol).

**Yield:** 22%; **¹H NMR (300 MHz, CDCl₃):** δ (ppm) 8.28 (dd, J = 7.2, 2.4 Hz, 2H), 7.39 (dd, J = 6.9, 2.1 Hz, 2H), 4.93 (s, 1H), 3.32 (br s, 1H), 3.13 (br s, 1H) 2.23-1.57 (m, 20H); **¹³C NMR (75 MHz, CDCl₃)** : δ (ppm) 155.7, 151.7, 145.5, 125.4, 122.0, 111.0, 75.6, 37.0, 33.2, 30.6, 27.1; **MS (ES⁺):** [M-NO₂]⁺ 415.2 (20).

### 1.6. Synthesis of compound (6): 5-hydroxydispiro[adamantane-2,3'-[1,2,4,5]tetraoxane-6',1"-cyclohexan]-3"-yl (4-nitrophenyl) carbonate

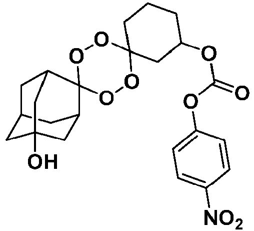

Compound **6** was prepared using the same method, but the starting material was compound **4** (0.151 g, 0.483 mmol). Purification by flash chromatography in Hexane:Ethyl acetate (1:1) isolated compound **6** as a white solid (0.0996 g, 0.208 mmol).
**Yield:** 43%; **¹H NMR (300 MHz, CDCl₃):** δ (ppm) 8.28 (dd, J = 7.2, 2.4 Hz, 2H), 7.36 (dd, J = 6.9, 2.4 Hz, 2H), 4.76-4.69 (m, 1H), 3.34-3.26 (m, 1H), 2.68-1.74 (m, 20H); **¹³C NMR (75 MHz, CDCl₃)** : δ (ppm) 155.4, 150.0, 145.5, 125.4, 121.9, 81.9, 73.9, 41.3, 39.8, 38.2, 34.7, 30.6, 29.9;

### 1.7. Synthesis of compound (8): 2-(hydroxymethyl)cyclohex-2-en-1-one

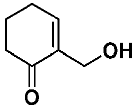

A 10 mL round-bottom flask was charged with barium hydroxide octahydrate (0.0079 g, 0.0250 mmol), dissolved in water/methanol (5:1; 2:0.5 mL) and N-methylpyrrolidine (5.5 µL, 0.0520 mmol) was added. Afterwards, formaldehyde (0.12 mL, 1.56 mmol) and compound **7** (0.10 mL, 1.04 mmol) were added successively to the previous solution and the reaction was left to stir at 0°C for 21 hours. The reaction mixture was quenched with a solution of hydrochloric acid (concentration: 1 mol/L) until pH = 3. Then, sodium bicarbonate saturated solution was added until pH around 7 and the resulting aqueous phase was extracted with dichloromethane (3x20 mL). The organic fractions were combined, dried over anhydrous sodium sulphate, filtered, and evaporated. Purification by flash chromatography using Hexane:Ethyl acetate (4:6) as eluent isolated compound **8** as a colourless oil (0.0706 g, 0.560 mmol).

**Yield:** 54%; **¹H NMR (300 MHz, CDCl₃):** δ (ppm) 6.93 (t, J = 4.2 Hz, 1H), 4.24 (q, J = 1.2 Hz, 2H), 2.48-2.35 (m, 4H), 2.32-2.12 (br s, 1H), 2.08-1.95 (m, 2H); **¹³C NMR (75 MHz, CDCl₃)** : δ (ppm) 200.6, 147.9, 138.2, 61.3, 38.2, 25.6, 22.7; **MS (ES⁺):** [M+H]⁺ 127 (75).

### 1.8. Synthesis of compound (9): (6,6-dihydroperoxycyclohex-1-en-1-yl)methanol

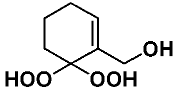

A 25 mL round-bottom flask was charged with compound **8** (0.0774 g, 0.614 mmol) and rhenium(VII) oxide (0.0171 g, 0.0353 mmol) in acetonitrile (3.7 mL). Then, hydrogen peroxide 50% (0.14 mL, 2.46 mmol) was added at room temperature and left to stir for 50 minutes. The mixture was filtered under vacuum through a silica plug and washed with ethyl acetate. The solution was concentrated, and compound **9** was isolated as a colourless oil (0.105 g, 0.598 mmol) .

**Yield:** 97%; **¹H NMR (300 MHz, (CD₃)₂CO):** δ (ppm) 9.97 (s, 1H), 9.65 (s, 1H), 4.26-4.13 (m, 1H), 4.02-3.89 (m, 1H), 3.71-3.62 (m, 1H), 2.49-1.25 (m, 2H), 2.01-1.62 (m, 4H); **¹³C NMR (75 MHz, (CD₃)₂CO):** δ (ppm) 145.1, 59.5, 37.8, 26.1, 23.8.

### 1.9. Synthesis of compound (10): 2"-(hydroxymethyl)dispiro[adamantane-2,3'-[1,2,4,5]tetraoxane-6',11"-cyclohexan]-2''-en-5-ol

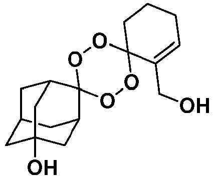

A 25 mL round-bottom flask was charged with 5-hydroxy-2-adamantanone (0.338 g, 2.03 mmol), rhenium(VII) oxide (0.0165 g, 0.034 mmol) in dichloromethane (4.0 mL) under inert atmosphere. Then, compound **9** (0.234 g, 1.33 mmol) in acetonitrile (4.0 mL) was added at room temperature and left to stir for 1 hour. The mixture was filtered under vacuum through a silica plug and washed with ethyl acetate. The solution was concentrated and purification by flash chromatography in Hexane:Ethyl acetate (2:8) isolated compound **10** as a white solid (0.132 g, 0.406 mmol).

**Yield:** 31%; **¹H NMR (300 MHz, CDCl₃):** δ (ppm) 4.68-4.59 (m, 1H), 3.24-3.15 (m, 1H), 2.49-2.39 (m, 2H), 2.16-1.68 (m, 18H); **MS (ES⁺):** [M+ACN+H]⁺ 366 (30), [M+Na]⁺ 347 (30).

### 1.10. Synthesis of compound (11): 5-hydroxydispiro[adamantane-2,3'-[1,2,4,5]tetraoxane-6',1"-cyclohexan]-2"-en-2"-yl)methyl (4-nitrophenyl) carbonate

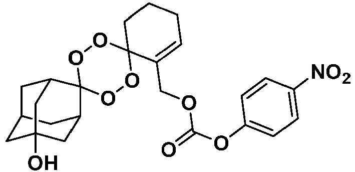

Compound **10** (0.126 g, 0.388 mmol) was dissolved in dichloromethane (1.53 mL) and, afterwards, *N,N-*diisopropylethylamine (0.20 mL, 1.17 mmol) and 4-dimethylaminopyridine (0.0510 g, 0.413 mmol) were added to the solution. At 0°C, 4-nitrophenyl chloroformate (0.162 g, 0.788 mmol) was added and the reaction was left to stir under inert atmosphere for 15 minutes, 0°C to room temperature for 10 minutes and at room temperature for 30 minutes. The reaction mixture was diluted in diethyl ether (30 mL), washed with sodium bicarbonate saturated solution (5x30 mL). The organic phase was washed with brine (1x25 mL), dried with anhydrous sodium sulphate, and afterward was filtered and concentrated. Purification by flash chromatography in Hexane:Ethyl acetate (1:1) isolated compound **11** as a white solid (0.0565 g, 0.115 mmol).

**Yield:** 30%; **¹H NMR (300 MHz, CDCl₃):** δ (ppm) 8.28 (dd, J = 7.0, 2.4 Hz, 2H), 7.36 (dd, J = 7.0, 2.4 Hz, 2H), 4.76-4.69 (m, 1H), 3.34-3.26 (m, 1H) 2.66-1.77 (m, 20H). **MS (ES⁺):** [M+ACN+Na]⁺ 553 (15), [M+NH₄]⁺ 507 (100).

### Example 2. Synthesis of compounds of the invention (IIa, IIb, IIc, IId and IIe)

### 2.1. Synthesis of compound (IIa): dispiro[adamantane-2,3'-[1,2,4,5]tetraoxane-6',1"-cyclohexan]-3"-yl (4-((7-methoxyquinolin-5-yl)amino)pentyl)carbamate

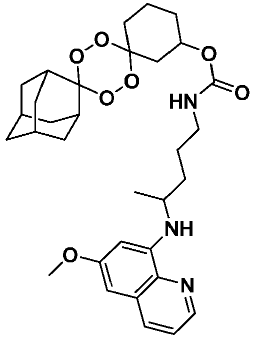

To a primaquine.diphosphate (0.042 g, 0.0922 mmol) solution in *N,N-*dimethylformamide (0.34 mL), triethylamine (12.0 µL, 0.0866 mmol) was added. After 30 minutes, 1-hydroxybenzotriazole hydrate (0.012 g, 0.0888 mmol) was added to the previous solution and compound 5 (0.0205 g, 0.0444 mmol) in N,N-dimethylformamide (0.34 mL). The mixture was kept stirring at room temperature overnight. Then, brine (20 mL) was added to the mixture and extracted with ethyl acetate (3x20 mL). The organic fractions were combined, washed with distilled water (1x20 mL), dried over anhydrous sodium sulphate, filtered and the solvent was removed under reduced pressure. The obtained residue was purified by preparative thin layer chromatography using Hexane:Ethyl acetate (7:3) as eluent. Compound **IIa** was isolated (0.0143 g, 0.024 mmol).

**Yield:** 55%; **¹H NMR (300 MHz, CDCl₃):** δ (ppm) 8.52 (dd, J = 4.2, 1.5 Hz, 1H), 7.91 (dd, J = 8.4, 1.5 Hz, 1H), 7.30 (dd, J = 8.4, 4.2 Hz, 1H), 6.32 (dd, J = 17.4, 2.4 Hz, 2H), 6.00 (br s, 1H), 4.82 (br s, 1H), 4.70 (br s, 1H), 3.89 (s, 3H), 3.62 (br s, 1H), 3.18 (br s, 3H), 2.81 (br s, 1H), 2.15-1.62 (m, 24H), 1.30 (d, J = 6.3 Hz, 3H); **MS (ES⁺):** [M+H]⁺ 582 (100).

### 2.2. Synthesis of compound (IIb): 1-(tert-butyl) 4-dispiro[adamantane-2,3'-[1,2,4,5]tetraoxane-6',1"-cyclohexan]-3"-yl) piperazine-1,4-dicarboxylate

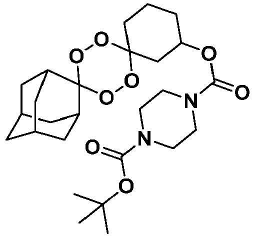

To a boc-piperazine (0.014 g, 0.0752 mmol) solution in *N,N-*dimethylformamide (0.27 mL), triethylamine (10.0 µL, 0.0684 mmol) was added. After 30 minutes, 1-hydroxybenzotriazole hydrate (0.0098 g, 0.0704 mmol) was added to the previous solution and compound **5** (0.0158 g, 0.0342 mmol) in N,N-dimethylformamide (0.27 mL). The mixture was kept stirring at room temperature overnight. Then, brine (20 mL) was added to the mixture and extracted with ethyl acetate (3x20 mL). The organic fractions were combined, washed with distilled water (1x20 mL), dried over anhydrous sodium sulphate, filtered and the solvent was removed under reduced pressure. The obtained residue was purified by preparative thin layer chromatography using Hexane:Ethyl acetate (8:2) as eluent. Compound **IIb** was isolated (0.0057 g, 0.011 mmol).

**Yield:** 33%; **¹H NMR (300 MHz, CDCl₃)** : δ (ppm) 4.91 (br s, 1H), 3.41 (m, 8H), 3.12 (br s, 1H), 3.00-2.56 (m, 1H), 2.31-1.55 (m, 20H), 1.46 (s, 9H).

### 2.3. Synthesis of compound (IIc): dispiro[adamantane-2,3'-[1,2,4,5]tetraoxane-6',1"-cyclohexan]-3"-yl (3-hydroxy-2-methyl-6-((2S,4S)-4,5,12-trihydroxy-4-(2-hydroxyacetyl)-10-methoxy-6,11-dioxo-1,2,3,4,6,11-hexahydrotetracen-2-yl)oxy)tetrahydro-2H-pyran-4-yl)carbamate

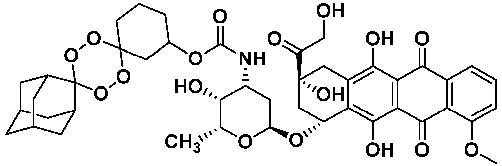

To a doxorubicin. hydrochloride (0.112 g, 0.193 mmol) solution in *N,N-*dimethylformamide (0.76 mL), triethylamine (27.0 µL, 0.193 mmol) was added. After 30 minutes, 1-hydroxybenzotriazole hydrate (0.027 g, 0.194 mmol) was added to the previous solution and compound **5** (0.0445 g, 0.0964 mmol) in *N,N-*dimethylformamide (0.76 mL). The mixture was kept stirring at room temperature for 3 hours. Then, brine (20 mL) was added to the mixture and extracted with ethyl acetate (3x20 mL). The organic fractions were combined, washed with distilled water (1x20 mL), dried over anhydrous sodium sulphate, filtered and the solvent was removed under reduced pressure. The obtained residue was purified by preparative thin layer chromatography using Dichloromethane:Methanol 4% as eluent. A red powder matching with the compound **IIc** was isolated (0.050 g, 0.058 mmol).

**Yield:** 60%; **¹H NMR (400 MHz, CDCl₃):** δ (ppm) 13.94 (d, J = 4.4 Hz, 1H), 13.18 (s, 1H), 8.00 (d, J = 7.6 Hz, 1H), 7.76 (t, J = 8.0 Hz, 1H), 7.37 (d, J = 8.4 Hz, 1H), 5.49 (br s, 1H), 5.25 (br s, 1H), 5.13-5.04 (m, 1H), 4.75 (s, 3H), 4.59-4.46 (m, 1H), 4.15-4.09 (m, 1H), 4.06 (s, 3H), 3.83 (br s, 1H), 3.67 (br s, 1H), 3.28-2.85 (m, 4H), 2.77 (br s, 1H), 2.68-2.37 (m, 1H), 2.36-2.26 (m, 1H), 2.24-2.08 (m, 2H), 2.04-1.57 (m, 21H), 1.28 (d, J = 6.8 Hz, 3H); **¹³C NMR (101 MHz, CDCl₃):** δ (ppm) 214.0, 187.1, 186.7, 161.1, 156.3, 155.7, 155.1, 135.9, 135.5, 133.7, 120.9, 119.9, 118.6, 111.6, 111.5, 110.8, 110.7, 100.9, 76.7, 69.8, 69.6, 67.4, 65.7, 56.8, 47.0, 37.0, 35.8, 34.1, 33.2, 31.1, 31.0, 30.3, 30.2, 29.8, 27.1, 17.0; **MS (ES⁺):** [M+Na]⁺ 888 (90); **HRMS (ESI):** m/z [M-H]⁻ calculated for C₄₄H₅₀NO₁₇: 864.3084, found: 864.3062.

### 2.4. Synthesis of compound (IId): 5-hydroxydispiro[adamantane-2,3'-[1,2,4,5]tetraoxane-6',1"-cyclohexan]-3"-yl (3-hydroxy-2-methyl-6-(((2S,4S)-4,5,12-trihydroxy-4-(2-hydroxyacetyl)-10-methoxy-6,11-dioxo-1,2,3,4,6,11-hexahydrotetracen-2-yl)oxy)tetrahydro-2H-pyran-4-yl)carbamate

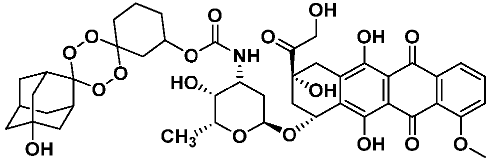

Compound **IId** was prepared using the same method, but the starting material was compound **6** (0.0310 g, 0.0649 mmol). Purification by preparative thin layer chromatography in Dichloromethane:Methanol 4% isolated compound **IId** as a red powder (0.0429 g, 0.049 mmol).

**Yield:** 75%; **¹H NMR (400 MHz, DMSO):** δ (ppm) 13.97 (s, 1H), 13.21 (s, 1H), 7.89-7.80 (m, 2H), 7.59 (d, J = 7.2 Hz, 1H), 6.56 (d, J = 8.0 Hz, 1H), 5.39 (s, 1H), 5.17 (s, 1H), 4.86 (d, J = 6.0 Hz, 2H), 4.84 (br s, 1H), 4.66 (d, J = 5.6 Hz, 1H), 4.55 (d, J = 5.6 Hz, 2H), 4.51 (br s, 1H), 4.11 (d, J = 6.8 Hz, 2H), 3.95 (s, 3H), 3.62 (br s, 2H), 3.44-3.37 (m, 2H), 3.02-2.82 (m, 4H), 2.76-2.60 (m, 1H), 2.45-1.51 (m, 19H), 1.09 (d, J = 6.4 Hz, 3H); **¹³C NMR (101 MHz, DMSO):** δ (ppm) 213.9, 186.5, 186.4, 176.7, 160.8, 156.1, 154.5, 154.1, 136.2, 135.5, 134.6, 134.1, 119.9, 119.7, 119.0, 110.8, 110.6, 100.4, 75.9, 75.0, 73.6, 69.8, 68.1, 66.7, 63.7, 56.6, 46.7, 40.9, 38.5, 36.5, 34.8, 34.7, 34.2, 32.1, 29.8, 29.7, 29.2, 17.0; **MS (ES⁺):** [M+2ACN+H]⁺ 964 (25), [M+CH3OH+H]⁺ 914 (45), [M+Na]⁺ 904 (30); **MS (ES⁻):** [M-H]⁻ 880 (35), [M+HCOOH-H]⁻ 925 (20).

### 2.5. Synthesis of compound (IIe): 5-hydroxydispiro[adamantane-2,3'-[1,2,4,5]tetraoxane-6',1"-cyclohexan]-2"-en-2"-yl)methyl (3-hydroxy-2-methyl-6-(((1R,3R)-3,5,12-trihydroxy-3-(2-hydroxyacetyl)-10-methoxy-6,11-dioxo-1,2,3,4,6,11-hexahydrotetracen-1-yl)oxy)tetrahydro-2H-pyran-4-yl) carbamate

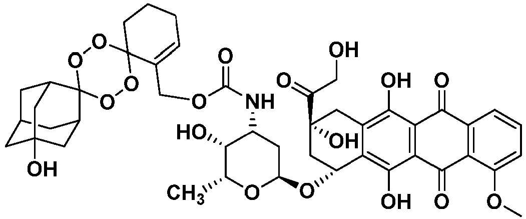

To a doxorubicin. hydrochloride (0.0743 g, 0.128 mmol) solution in N,N-dimethylformamide (0.50 mL), triethylamine (18.0 µL, 0.128 mmol) was added. After 30 minutes, 1-hydroxybenzotriazole hydrate (0.0183 g, 0.135 mmol) was added to the previous solution and compound **11** (0.0312 g, 0.064 mmol) in N,N-dimethylformamide (0.50 mL). The mixture was kept stirring at room temperature for 7 hours. Then, brine (20 mL) was added to the mixture and extracted with ethyl acetate (3x20 mL). The organic fractions were combined, washed with distilled water (1x20 mL), dried over anhydrous sodium sulphate, filtered and the solvent was removed under reduced pressure. The obtained residue was purified by preparative thin layer chromatography (TLC) using Dichloromethane:Methanol 4% as eluent. Compound **IIe** was isolated as a red powder (0.0415 g, 0.046 mmol).

**Yield:** 73%; **¹H NMR (400 MHz, DMSO):** δ (ppm) 13.95 (s, 1H), 13.20 (s, 1H), 7.90-7.76 (m, 2H), 7.58 (d, J = 7.6 Hz, 1H), 6.56 (d, J = 8.0 Hz, 1H), 5.37 (s, 1H), 5.16 (s, 1H), 4.91-4.81 (m, 2H), 4.73-4.61 (m, 1H), 4.60-4.45 (m, 4H), 4.11 (d, J = 7.2 Hz, 1H), 3.94 (s, 3H), 3.62 (br s, 2H), 3.39 (br s, 2H), 3.04-2.75 (m, 4H), 2.40-1.50 (m, 20H), 1.41 (d, J = 12 Hz, 1H), 1.09 (d, J = 6.4 Hz, 3H); **¹³C NMR (101 MHz, DMSO):** δ (ppm) 213.9, 186.5, 186.4, 176.7, 160.8, 156.1, 154.5, 154.1, 136.2, 135.5, 134.6, 134.0, 119.9, 119.7, 119.0, 110.7, 110.6, 100.4, 75.9, 75.0, 73.5, 69.8, 68.1, 66.7, 63.7, 56.6, 46.7, 40.9, 38.5, 36.5, 34.8, 34.7, 34.2, 32.1, 29.8, 29.7, 29.2, 17.0; **MS (ES⁺):** [M+H]⁺ 894 (35), [M+Na]⁺ 916 (70); **MS (ES⁻):** [M-H]⁻ 892 (40).

### Example 3 - Mechanistic proof-of-concept of the first drug delivery system

A solution of compound **IIc** (0.009 mmol) in dichloromethane was added to a schlenk tube, under inert atmosphere, charged with iron(II) bromide (2 equivalents) and TEMPO (2 equivalents) in acetonitrile. The reaction was left to stir at room temperature for 24 hours. The reaction mixture was diluted in ethyl acetate (10 mL), washed with distilled water (1x10 mL) and brine (1x10 mL). The organic fraction was dried over anhydrous sodium sulphate, filtered and the solvent was removed under reduced pressure. The residue was analysed by UHPLC-MS. The mobile phase consisted of millipore water containing 0.1% formic acid (A) and acetonitrile (B), at a flow rate of 0.30 mL min⁻¹. A gradient elution was applied, consisting of 10% B for 1 minutes; from 10 to 95% B for 5 minutes and 95% B for 4 minutes, finally returning to the initial conditions (10% B) for 5 minutes.

Tetraoxane reaction with a Fe(II) source and TEMPO was performed for compound **IIc** to confirm tetraoxane fragmentation and, more importantly, that spontaneous β-elimination of the cyclohexanone derivative occurs to liberate doxorubicin.

The UHPLC-MS analysis of this reaction detected two TEMPO adducts on positive ([M+H]⁺= 324) and negative mode ([M-H]⁻= 322; [M-H]⁻= 855 and [M+CN-H]⁻= 881), for the two possibilities of coordination with Fe(II), which confirms tetraoxane fragmentation with formation of carbon-centred radicals (Scheme 5). Additionally, 2-adamantanone ([M+K]⁺= 189) and the cyclohexanone derivative ([M-H]⁻= 682) were also identified, in accordance with the proposed mechanism. Importantly, doxorubicin was also detected ([M+H]⁺= 544; [M-H]⁻= 542), confirming API release by β-elimination of the cyclohexanone derivative (Scheme 6).

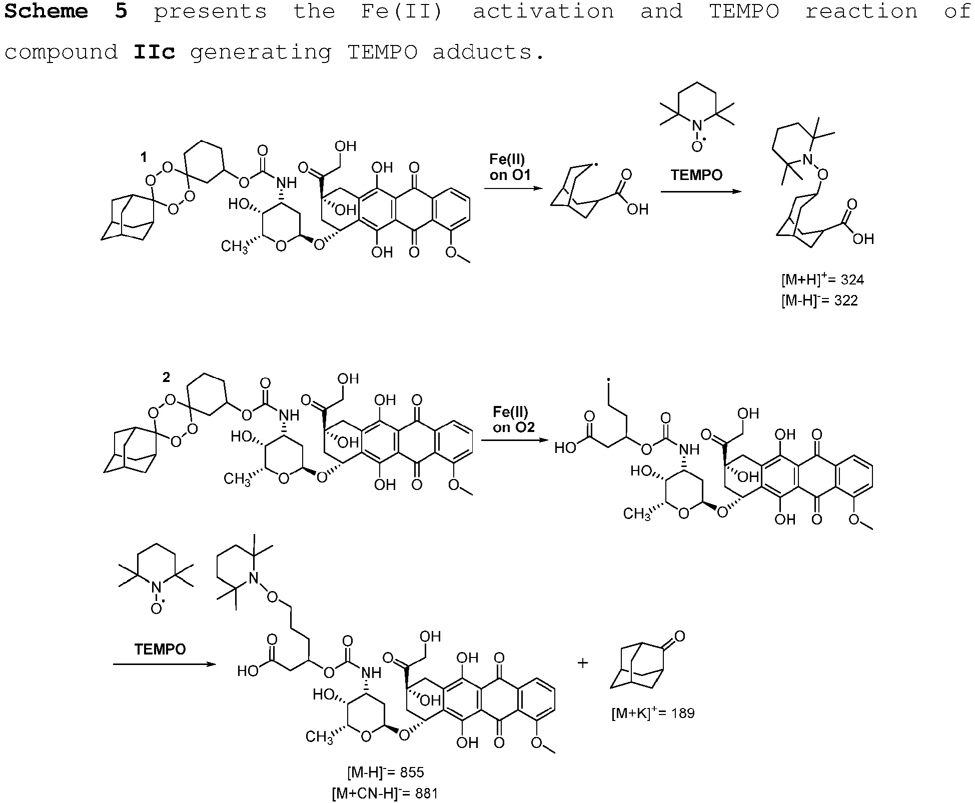

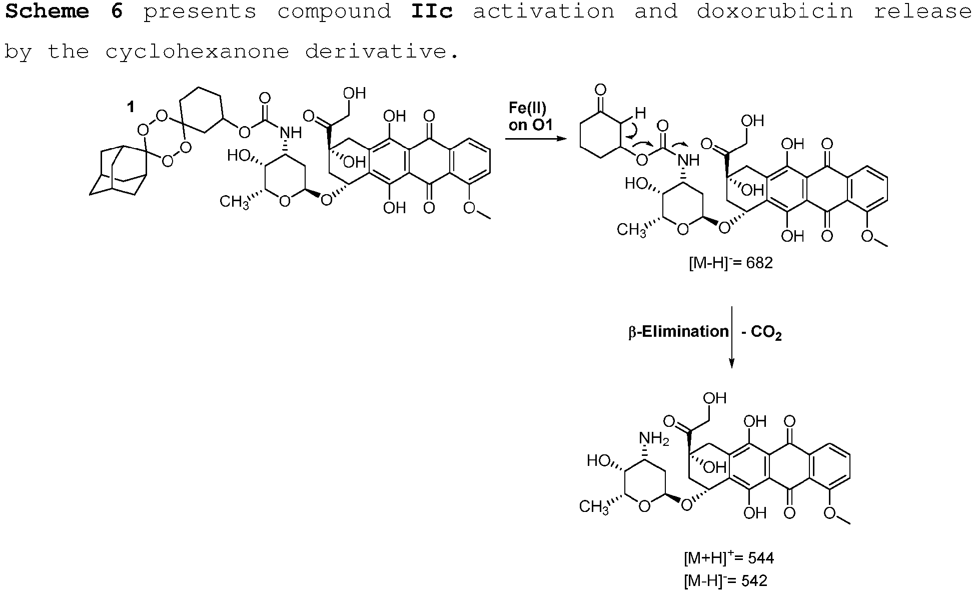

### Example 4 - Mechanistic proof-of-concept of the second drug delivery system

To validate the ferrous iron activation and TEMPO trapping of the second drug delivery system, the following protocol was performed. A solution of compound **IIe** (0.0077 g, 0.009 mmol) in dichloromethane was added to a schlenk tube, under inert atmosphere, charged with iron(II) bromide (2 equivalents) and TEMPO (2 equivalents) in tetrahydrofuran. The reaction was left to stir at room temperature for 24 hours. The reaction mixture was diluted in ethyl acetate (10 mL), washed with distilled water (1x10 mL) and brine (1x10 mL). The organic fraction was dried over anhydrous sodium sulphate, filtered and the solvent was removed under reduced pressure. The residue was analysed by UHPLC-MS. The mobile phase consisted of millipore water containing 0.1% formic acid (A) and acetonitrile (B), at a flow rate of 0.30 mL min⁻¹. A gradient elution was applied, consisting of 30% B for 1 minutes; from 30 to 95% B for 5 minutes and 95% B for 4 minutes, finally returning to the initial conditions (30% B) for 5 minutes.

To validate the ferrous iron activation and N-acetylcysteine (NAC) Michael addition of the second drug delivery system, the following protocol was performed. 250 µL of a 10 mM stock solution of compound **IIe** in tetrahydrofuran was added to a schlenk tube, under inert atmosphere, charged with iron(II) bromide (2 equivalents) and NAC (1.5 equivalents) in 250 µL of millipore water. Then, 100 µL of sodium hydroxide (concentration: 6.25 mol/L) was added to assure basic pH. The reaction was left to stir at room temperature for 24 hours. The reaction mixture was diluted in distilled water (10 mL) and extracted with ethyl acetate (10 mL). The aqueous fraction was acidified with hydrochloric acid (concentration: 1 mol/L) and extracted with ethyl acetate (2x10 mL). The aqueous phase was neutralized with sodium hydroxide (concentration: 6.25 mol/L) and extracted with ethyl acetate (2x10 mL). The organic fractions were combined, dried over anhydrous sodium sulphate, filtered and the solvent was removed under reduced pressure. The residue was analysed by UHPLC-MS. The mobile phase consisted of millipore water containing 0.1% formic acid (A) and acetonitrile (B), at a flow rate of 0.30 mL min⁻¹. A gradient elution was applied, consisting of 30% B for 1 minutes; from 30 to 95% B for 5 minutes and 95% B for 4 minutes, finally returning to the initial conditions (30% B) for 5 minutes.

Compound **IIe** reacted with an iron source and TEMPO trapping to confirm tetraoxane fragmentation and, more importantly, that doxorubicin was not released under these conditions.

The UHPLC-MS analysis of this reaction detected two TEMPO adducts on positive mode ([M+H]⁺= 340; [M+K]⁺= 907), for the two possibilities of coordination with Fe(II), which confirms tetraoxane fragmentation with formation of carbon-centred radicals (Scheme 7).

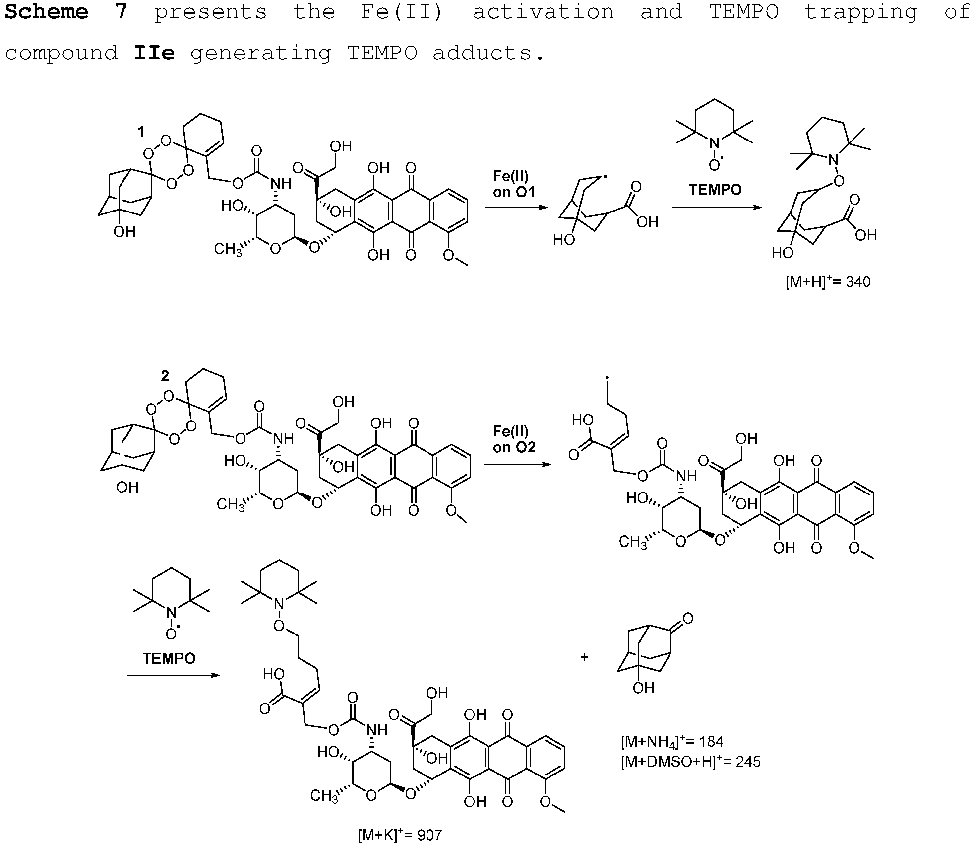

Additionally, 5-hydroxy-2-adamantanone ([M+NH4]⁺= 184; [M+DMSO+H]⁺= 245) and the cyclohex-2-enone derivative ([M+K]⁺= 734; [M+DMSO+H]⁺= 774) were also identified, in accordance with the proposed mechanism (Scheme 7 and 8). Finally, in this experiment doxorubicin was not detected.

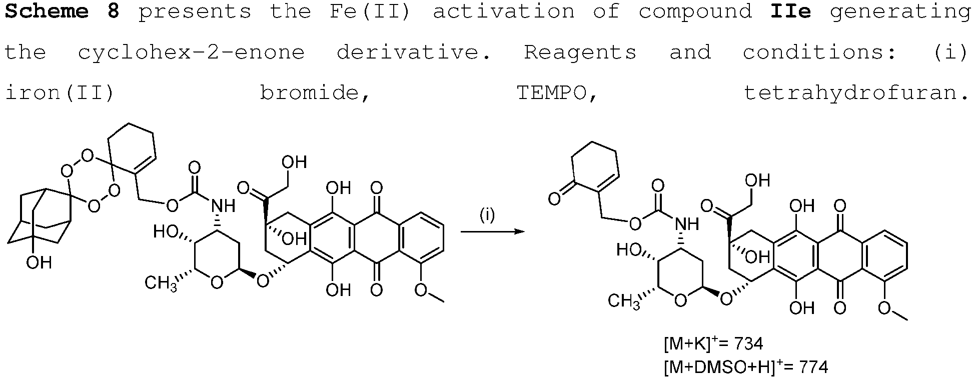

Compound **IIe** reacted with a Fe(II) source and N-acetylcysteine (NAC) to confirm the mechanism of the second drug delivery system. The UHPLC-MS analysis of this reaction detected one carbon-centred radical (Scheme 9) on positive mode ([M+H]⁺= 713 and [M+Na]⁺= 735), confirming tetraoxane fragmentation upon reaction with Fe(II). 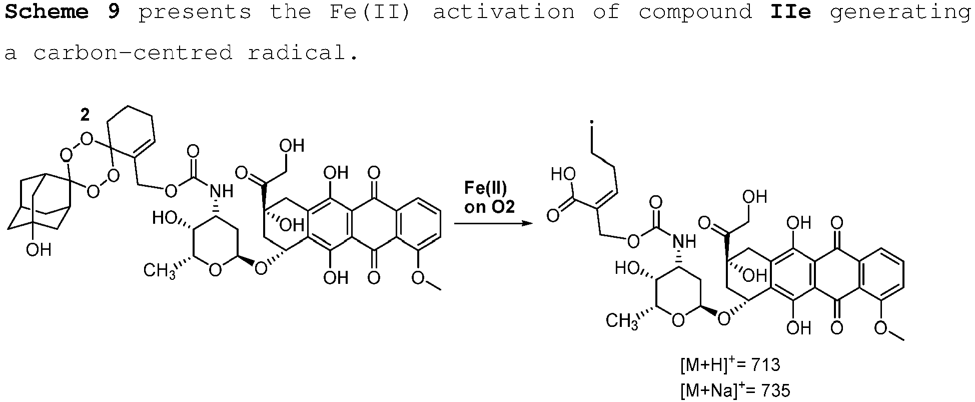 Additionally, the cyclohex-2-enone derivative ([M+Na]⁺= 718 and [M+K]⁺= 734) was also detected, in accordance with the first step of activation of the second drug delivery system. Finally, release of doxorubicin was detected, validating the mechanism of action for the second drug delivery system because Michael addition of NAC to the cyclohex-2-enone derivative occurred (Scheme 10).

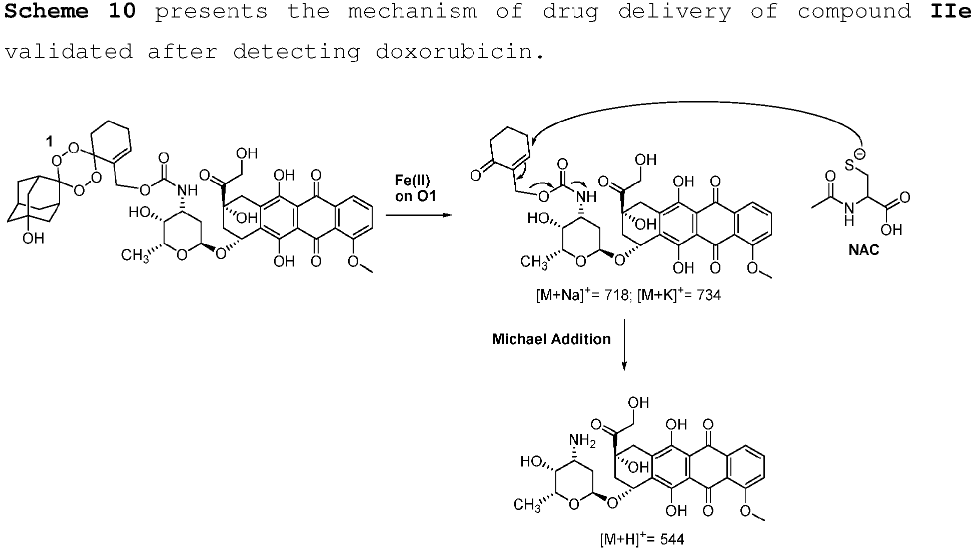

### Example 5. Evaluation of biological activity of compounds of formulae II against malaria

### Sample preparation

Compounds were dissolved in RPMI-1640 with L-glutamine (Biowest^{™}) supplemented with AlbuMAXII (Gibco^{™}), herein after referred to as RPMIc, to obtain an intermediate solution of 100 µM.

### P. falciparum in vitro culture

Laboratory-adapted *P*. *falciparum* strain 3D7, a chloroquine and mefloquine sensitive strain, was continuously cultured as described elsewhere (Machado et. al, Ann. Clin. Med. Microbio. 2016, 2, 1010). Parasites were cultivated in 5% haematocrit, 37°C and atmosphere with 5% of CO₂. AlbuMAXII (Gibco^{™}) at 0,5% was used as a substitute of human serum in the culture medium.

### Antimalarial activity determined using the Whole-Cell SYBR Green I assay

Staging and parasitemia were determined by light microscopy of Giemsa-stained thin blood smears. Anti-malarial activity was determined using the SYBR Green I assay (Machado et. al, Ann. Clin. Med. Microbio. 2016, 2, 1010 and Lobo et. al, Malar. J. 2018, 17, 1). Briefly, asynchronous parasites were cultivated for 72 hours in the presence of a 1:3 serial dilution of each compound. Fluorescence intensity was measured with a multi-mode microplate reader (Triad, Dynex Technologies), with excitation and emission wavelengths of 485 and 535 nm, respectively, and analysed by nonlinear regression using GraphPad Prism to determine IC₅₀ values.

Compounds **IIa** and **IIb** were potent against chloroquine-sensitive 3D7 strain of *Plasmodium falciparum.* Compound **IIa** and **IIb** have a IC₅₀ of 32.82 nM and 11.38 nM, respectively (Table 1). Both compounds were as potent as chloroquine (CQ), an antimalarial used in the clinic (Table 1).

**Table 1 - Antimalarial activity (IC₅₀) of the compounds against Plasmodium falciparum.**

| **COMPOUND** | **Mean*** | **SD** |
|---|---|---|
| **IIa** | 32.82 | 13.37 |
| **IIb** | 11.38 | 1.6 |
| **CQ** | 19.4 | 2.2 |

| | | |
|---|---|---|
| *Results in nanomolar (nM) from at least 3 independent assays, each in triplicate. SD, standard deviation. CQ, chloroquine. | | |

### Example 6. Evaluation of biological activity of compounds of formulae II in cancer treatment

PC3 human prostate adenocarcinoma, MCF7 human breast adenocarcinoma, HCT116 human colorectal carcinoma and U251 human glioblastoma were obtained from American Type Culture Collection (ATCC).

Cell lines were cultured under adherent conditions in Roswell Park Memorial Institute (RPMI) 1640 (PC3), Dulbecco's modified Eagle's medium (DMEM; MCF7 and U251), McCoy's 5A (HCT116), all supplemented with 10% (v/v) heat-inactivated fetal bovine serum (FBS) and 1% (v/v) antibiotic/antimycotic solution (all from Gibco, ThermoFisher Scientific, Paisley, UK). Additionally, MCF7 media was supplemented with 1% (v/v) glutamax (Gibco); U251 supplemented with 1% (v/v) glutamax and 1% (v/v) 4-(2-hydroxyethyl)-1-piperazineethanesulfonic acid (HEPES) buffer (both from Gibco). All cell lines were cultured at 37°C under a humidified atmosphere of 5% CO₂ (HeraCell 150i CO₂ incubator, ThermoFisher Scientific).

To quantitatively assess the compounds inhibitory potency, their half maximal inhibitory concentration (IC₅₀) was determined through dose-response curves. Cell lines were plated in 96-well plates at a cellular density of 5000 cells/well, and 24 hours after, cells were treated with compounds **IIc, IId, IIe,** doxorubicin, compound **3** and compound **10** (concentrations ranging from 1.5x10⁻³ ρM to 1 mM). Following 72 hours of incubation, cell metabolic activity assessed by determination of MTS metabolism. Viable cells (metabolically active cells) will metabolize the MTS into formazan and this molecule is quantified by absorbance. The higher the amount of formazan more cells survived after exposure to the compounds while the lower the amount of formazan more cells are not metabolically active.

For all assays, cellular metabolic activity was measured using CellTiter 96^{®} Aqueous Non-Radioactive Cell Proliferation Assay (MTS; Promega, Madison, WI, USA) and absorbance signal recorded using a GloMax^{®}-MultiDetection System (Promega). IC₅₀ determination and all statistical analysis was preformed using GraphPad Prism 8.0.2. software (San Diego, CA, USA). All data are expressed as mean ± SEM from at least three independent experiments. According to the normality of values distribution, assessed with Shapiro-Wilk test, Ordinary One-Way ANOVA or Kruskal-Wallis test, followed by Bonferroni or Dunn's multiple comparison test were used to evaluate differences among sample groups. A p-value inferior to 0.05 was considered significant.

All compounds were tested alone and in suitable doses on a panel of cancer cell lines with the results shown in Table 2.

As it is possible to observe from Table 2, compound **IIc** was potent in all cell lines in the micromolar range, except for the U251 cell line, which was in the submicromolar range. Moreover, compound **IIc** was less potent than doxorubicin alone.

Compound **IId** was also tested on the cancer cell lines used previously (U251, PC3, MCF7 and HCT116). Despite their difference in terms of lipophilicity, compound **IId** was also potent in the micromolar range and less potent than doxorubicin.

Compound **(3)** was tested as a control to demonstrate that the potency of compounds **IIc** and **IId** on cancer cells relies on the API coupled to compound **(3).** This compound was chosen since compound **(3)** represents the central structure of compounds **IIc** and **IId.**

Compound **(3)** was not active on the tested cell lines.

These results show that the potency of compounds **IIc** and **IId** relies on the API coupled in the system and not on the tetraoxane structure.

Compound **IIe** was potent in the micromolar range in all cancer cell lines. Compound **IIe** was less potent than doxorubicin in all cancer cell lines evaluated.

Compound **(10)** was tested as a control to demonstrate that the potency of compound **IIe** on cancer cells relies on the API coupled to compound **(10).** This compound was chosen since compound **10** represents the central structure of compound **IIe.**

Compound **(10)** was not active in all the cell lines tested (Table 2). Hence, these results prove that the tetraoxane scaffold does not possess cytotoxicity and the contributor of the activity of compound **IIe** is the API coupled in the system, in this case, doxorubicin.

**Table 2 - IC₅₀ of the tested compounds in all cell lines. Data represent IC₅₀ values of three independent experiments and respective confidence interval**

| **Compounds** | **U251 (95% C.I.)** | **PC3 (95% C.I.)** | **HCT116 (95% C.I.)** | **MCF7 (95% C.I.)** |
|---|---|---|---|---|
| **IIc** | 0.25 (0.08-0.77) | 1.37 (0.38-6.23) | 5.11 (0.33-N.D.) | 1.65 (1.06-2.47) |
| **IId** | 5.21 (1.36-45.99) | 3.07 (1.5-5.9) | 3.32 (2.39-4.99) | 6.69 (3.68-12.27) |
| **IIe** | 13.17 (8.00-29.62) | 14.31 (N.D.-25.31) | 3.52 (2.50-5.19) | 8.45 (6.17-11.42) |
| **3** | N.A. | N.A. | N.D. | N.D. |
| **10** | N.A. | N.A. | N.A. | N.A. |
| **Doxorubicin** | 0.011 (N.D.) | 0.12 (0.04-0.28) | 0.05 (0.02-0.18) | 0.17 (0.11-0.27) |

| | | | | |
|---|---|---|---|---|
| Results in micromolar (µM) from at least 3 independent assays. C. I.: confidence interval; N. D.: not determined; N. A.: not active. | | | | |

### Example 7. Evaluation of the effect of compounds of formulae II in toxicity for non-tumoral cells

To evaluate the toxicity effect of doxorubicin, compounds **IIc, IId, IIe** and doxorubicin were tested on a non-tumorigenic cell line (AML12) and the results are shown in Table 3. AML12 murine immortalized hepatocyte cell line was obtained from European Collection of Authenticated Cell Cultures (Porton Down, UK). AML12 cell line was cultured under adherent conditions in DMEM/Nutrient mixture F-12 (Gibco, ThermoFisher Scientific, Paisley, UK), supplemented with 10% (v/v) heat-inactivated fetal bovine serum (FBS; Gibco), 1% (v/v) antibiotic/antimycotic solution (Gibco) 1% (v/v) insulin-transferrin-selenium (Gibco) and 40 ng/mL of dexamethasone (Sigma-Aldrich, St. Louis, MO, USA). This cell line was cultured at 37°C under a humidified atmosphere of 5% CO₂ (HeraCell 150i CO₂ incubator, ThermoFisher Scientific). AML12 cells were plated in 96-well plates at a cellular density of 5000 cells/well, and 24 hours after, cells were treated with compound **IIc, IId, IIe** and doxorubicin (concentrations ranging from 1.5x10⁻³ ρM to 1 mM). Cellular metabolic activity was measured after 72 hours using CellTiter 96^{®} Aqueous Non-Radioactive Cell Proliferation Assay (MTS; Promega, Madison, WI, USA) and absorbance signal recorded using a GloMax^{®}-MultiDetection System (Promega). IC₅₀ determination and all statistical analysis was preformed using GraphPad Prism 8.0.2. software (San Diego, CA, USA). All data are expressed as mean ± SEM from at least three independent experiments. According to the normality of values distribution, assessed with Shapiro-Wilk test, Ordinary One-Way ANOVA or Kruskal-Wallis test, followed by Bonferroni or Dunn's multiple comparison test were used to evaluate differences among sample groups. A p-value < 0.05 was considered significant.

From Table 3 it is possible to observe that both compounds **IIc** and **IId** were able to induce reduced toxicity than doxorubicin. Interestingly, compound **IIc** was 10 times less toxic when compared with doxorubicin. However, AML12 cells were highly resistant to compound **IId,** with a IC₅₀ ratio of 4294.

In conclusion, the first drug delivery system according to the present invention is able to reduce the toxicity of doxorubicin.

From Table 3 it is possible to observe that compound **IIe** was less toxic in non-tumorigenic cells than doxorubicin. Interestingly, compound **IIe** was 180-fold less toxic when compared with doxorubicin. So, the second drug delivery system can reduce the toxicity associated with doxorubicin.

**Table 3 - IC₅₀ of the tested compounds in AML12 cell line. Data represent IC₅₀ values of three independent experiments and respective confidence interval**

| **Compounds** | **AML12 (95% CI)** | **Selectivity (IC₅₀Compound II/IC₅₀ Doxorubicin)** |
|---|---|---|
| **IIc** | 1.83 (0.98-3.2) | 10.64 |
| **IId** | 730 (0.15-N.D.) | 4294 |
| **IIe** | 30.49 (11.27-878.2) | 179 |
| **Doxorubicin** | 0.17 (0.11-0.26) | - |

| | | |
|---|---|---|
| Results in micromolar (µM) from at least 3 independent assays. C. I.: confidence interval; N. D.: not determined. | | |

### Example 8. Evaluation of biological activity dependent on iron

For iron competition assays, HCT116 cells were incubated with 1 µM DFO (Figure 1 column #2)), 10 µM compound **IId** (Figure 1 column #3)) and 10 µM compound **IId** together with 1 µM DFO (Figure 1 column #4)). Moreover, HCT116 cells were incubated with 1 µM DFO (Figure 2 column #2)), 10 µM compound **IIe** (Figure 2 column #3)) and 10 µM compound **IIe** together with 1 µM DFO (Figure 2 column #4)). Following 24 hours of incubation, cellular metabolic activity was determined. Cellular metabolic activity was measured using CellTiter 96^{®} Aqueous Non-Radioactive Cell Proliferation Assay (MTS; Promega, Madison, WI, USA) and absorbance signal recorded using a GloMax^{®}-MultiDetection System (Promega). IC₅₀ determination and all statistical analysis was performed using GraphPad Prism 8.0.2. software (San Diego, CA, USA). All data are expressed as mean ± SEM from at least three independent experiments. According to the normality of values distribution, assessed with Shapiro-Wilk test, Ordinary One-Way ANOVA or Kruskal-Wallis test, followed by Bonferroni or Dunn's multiple comparison test were used to evaluate differences among sample groups. A p-value < 0.05 was considered significant.

Compound **IId** was incubated with an iron chelator (deferoxamine mesylate - DFO) of ferrous iron in HCT116 cells. A significant reduction was detected in the potency of compound **IId** (Figure 1 column #4)) when compared with compound **IId** alone (Figure 1 column #3)). Thus, cell death by compound **IId** is iron-dependent.

Compound **IIe** was incubated with the iron chelator DFO in HCT116 cells. A small reduction (7%) was detected in the potency of compound **IIe** (Figure 2 column #4)) when compared with compound **IIe** alone (Figure 2 column #3)). Probably, compound **IIe** is activated by Fe(II) as described, but the major contributor of the cytotoxicity is related with the reaction of the cyclohex-2-enone derivative with glutathione (Michael addition), allowing the release of the API by elimination (see Example 4). The cyclohex-2-enone derivative possesses an alkene group that only becomes available after tetraoxane activation by Fe(II), which allows to improve the selectivity when compared with the first drug delivery system.

### Example 9. Evaluation of biological activity of compounds of formulae II as tumour markers

### Quantum yields determination

Ultraviolet (UV) spectra were traced in Thermo Scientific Evolution 201 UV-visible spectrophotometer. Fluorescence measurements were done on a SHIMADZU Spectro fluorophotometer RF-6000 instrument. Fluorescence quantum yields were measured with Fluorescein in 0.1 M sodium hydroxide as a standard (Φf = 0.95). All solvents were of analytical reagent grade and were purchased from Alfa Aesar or Sigma-Aldrich. Doxorubicin has fluorescence properties. Dilute solutions of compounds **IIc, IId** and doxorubicin in water with 30% dimethyl sulfoxide (DMSO) were prepared. The quantum yield of doxorubicin was 2.8% and the quantum yield of compounds **IIc** and **IId** was below 1%.

### Fluorescence imaging for compound cellular localization

HCT116 cells were plated in 35 mm dishes at 2.5x10⁵ cells/ml cellular density over a glass coverslip. After 24 hours of plating, cells were incubated for another 24 hours with system control (DMSO) or 1 µM of compounds **IId** or doxorubicin. Cells were gently washed with Phosphate Buffered Saline (PBS) 1x and fixed with 4% paraformaldehyde (PFA), pH 7.4 at room temperature. Fixed cells were incubated with Hoechst dye 33258 (5 µg/mL) for 10 minutes in the dark. Cells were washed 3x with PBS 1x and mounted into a glass slide with 7 µL Mowiol^{®}. Images were acquired with a Zeiss Axio Scope.A1 microscope coupled with an AxioCam HR R3 camera and images analysed with Zen Software 2012 Blue Edition (Carl Zeiss Microscopy, GmbH).

Doxorubicin photoluminescence properties are ablated in the drug delivery system of formula II (quantum yield below 1%). Therefore, next we explored the cellular localization of compound **IId** in cells. Interestingly, fluorescence imaging in HCT116 cells showed that after 24 hours of incubation, doxorubicin accumulated in the nucleus (Figure 3 BC and BD) while compound **IId** was activated and released doxorubicin in the cytosol (Figure 3 CC and CD), where the labile iron pool is elevated.

### Example 10 - In vivo validation of the first drug delivery system

The antitumor effect of compound **IId** was tested in a xenograft murine model. Immunocompromised Foxn1^{nu/nu} mice received a subcutaneous injection of a colorectal cancer cell line (2x10⁶ HT-29 cells) suspended in PBS (phosphate buffered saline) solution (100 µL) in the right flank. When tumour masses became palpable, treatment started. Four groups, with five mice per group, were established: negative control group - mice that did not receive any treatment; vehicle control - mice that received cremophor in PBS (phosphate buffered saline) solution (the solvent used for solubilizing compound **IId**); **IId** - mice that received the compound under study; doxorubicin - mice that received doxorubicin solubilized in PBS (phosphate buffered saline) solution. All tested formulations were intravenously administered in the tail vein in a total number of eight injections with a therapeutic dose of 2 mg/kg of body weight. The doses administered to mice were established by a ratio between the mass of compound and body weight of the animal. Tumour size was regularly monitored using a digital calliper. Respective tumour volumes were calculated according to the formula: V (mm³) = (L×W²)/2, where L and W represent the longest and shortest axis of the tumour, respectively. Relative tumour volume was also determined for each animal: ratio between volumes at the indicated day and volume at the beginning of treatment. Body weight was also regularly evaluated.

Mice treated with compound **IId** and doxorubicin had a higher reduction of the tumour mass when compared with negative control group and the vehicle control (Figure 4). Also, the reduction of the tumour mass was higher for mice treated with compound **IId** (Figure 4). The molecular weight of compound **IId** is 30% higher than the molecular weight of doxorubicin. Therefore, the number of moles of doxorubicin released from compound **IId** was 30% lower when compared with the number of moles of the administration of doxorubicin alone. Possibly, an experiment where the animals are administered with the same number of moles of both compounds can lead to a higher difference in the reduction of the tumour mass between compound **IId** and doxorubicin.

## Claims

1. A **compound** of formula I, including enantiomers and racemic mixtures thereof, wherein: 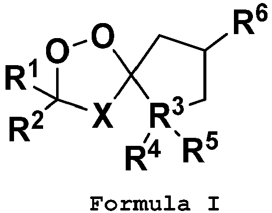
R¹ and R² are independently hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl,
• R¹ and R² may be joined to form a substituted or unsubstituted cycloalkyl and substituted or unsubstituted heterocycloalkyl,
• X is -CH₂O-, -OC, H₂- or -O-O-
• R³ is -C(R⁴)(R⁵)-, -CH(R⁴)-CH(R⁵)- or -C(R⁴)=C(R⁵)-
• R⁴ is hydrogen, -OH, -CH₂OH, -CH₂O(C=O)A or -CH₂O(C=S)A, wherein A is independently one or more API, proteins and antibodies,
• R⁵ and R⁶ are independently hydrogen, -OH, -O(C=O)A or - O(C=S)A, wherein A is independently one or more API, proteins or antibodies,
**and such compound is characterized by being represented by any of the following formulae below:**
**the formula IIa, wherein,**
**R¹** is adamantyl
**R²** is adamantyl
**X** is -O-O-
**R³** is -CH(R⁴)-CH(R⁵)-
**R⁴** is hydrogen
**R⁵** is (4-((7-methoxyquinolin-5-yl)amino)pentyl) carbamoyloxy and
**R⁶** is hydrogen 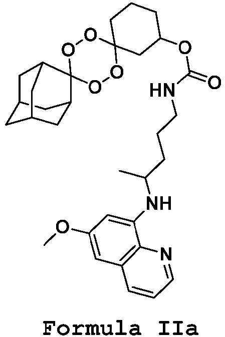 **the formula IIb, wherein**
**R¹** is adamantyl
**R²** is adamantyl
**X** is -O-O-
**R³** is -CH(R⁴)-CH(R⁵)-
**R⁴** is hydrogen
**R⁵** is 1-(tert-butyl) piperazine-1,4-dicarboxylate and
**R⁶** is hydrogen 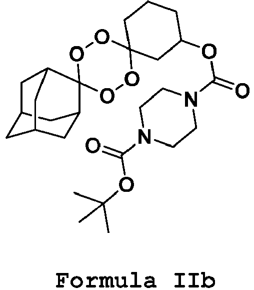 **the formula IIc, wherein:**
**R¹** is adamantyl
**R²** is adamantyl
**X** is -O-O-
**R³** is -CH(R⁴)-CH(R⁵)-
**R⁴** is hydrogen
**R⁵** is (3-hydroxy-2-methyl-6-((2S,4S)-4,5,12-trihydroxy-4- (2-hydroxyacetyl)-10-methoxy-6,11-dioxo-1,2,3,4,6,11- hexahydrotetracen-2-yl)oxy)tetrahydro-2H-pyran-4- yl)carbamoyloxy and
**R⁶** is hydrogen 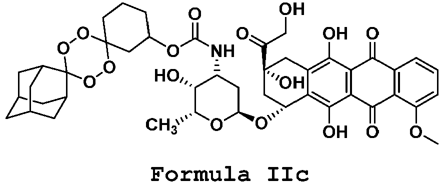 **the formula IId, wherein:**
**R¹** is 5-hydroxy adamantyl
**R²** is 5-hydroxy adamantyl
**X** is -O-O-
**R³** is -CH(R⁴)-CH(R⁵)-
**R⁴** is hydrogen
**R⁵** is (3-hydroxy-2-methyl-6-(((2S,4S)-4,5,12-trihydroxy-4- (2-hydroxyacetyl)-10-methoxy-6,11-dioxo-1,2,3,4,6,11- hexahydrotetracen-2-yl)oxy)tetrahydro-2H-pyran-4-yl) carbamoyloxy and
**R⁶** is hydrogen 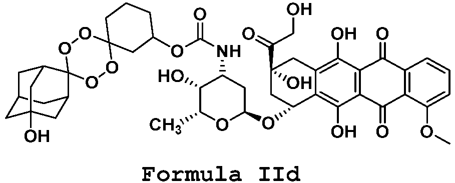 **the formula IIe, wherein:**
**R¹** is 5-hydroxy adamantyl
**R²** is 5-hydroxy adamantyl
**X** is -O-O-
**R³** is -C(R⁴)=C(R⁵)-
**R⁴** is (3-hydroxy-2-methyl-6-(((1R,3R)-3,5,12-trihydroxy-3-(2-hydroxyacetyl)-10-methoxy-6,11-dioxo-1,2,3,4,6,11-hexahydrotetracen-1-yl)oxy)tetrahydro-2H-pyran-4-yl)carbamoyloxy
**R⁵** is hydrogen and
**R⁶** is hydrogen

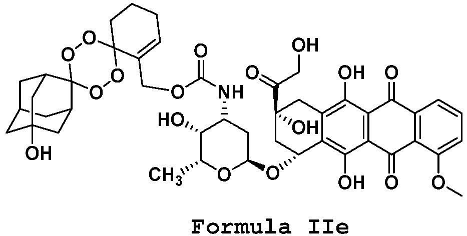

2. A **drug delivery system characterized by** being based on tetraoxanes compounds of **formula IIa, IIb, IIc, IId** and **IIe,** as described in claim 1, being said compounds appropriately modified to release active pharmaceutical ingredients (API), wherein **compounds IIa or IIb are coupled with one or more antimalarial API, compounds IIc, IId or IIe are coupled with one or more antitumour API, or compounds IIc, IId or IIe are coupled with one or more tumour marker APIs.**

3. A **drug delivery system** according to claim 2 **characterized by** the **antimalarial** APIs, are selected from primaquine and boc-piperazine, atovaquone, quinine sulphate, sulphonamides (sulphadoxine and sulphamethoxypyridazine) and falcipain inhibitors (dipeptidyl vinyl sulfones and peptidomimetic pyrimidine nitriles), and combinations or derivatives thereof.

4. A **drug delivery system** according to claim 2 **characterized by** the **antitumour** APIs, are selected from doxorubicin, alectinib, afatinib, abiraterone acetate, azacitidine, axitinib, bendamustine, brigatinib, bosutinib, bicalutamide, calicheamicin, capecitabine, carfilzomib, combretastatin, ceritinib, cladribine, clofarabine, cobimetinib, crizotinib, cryptophycin, cytarabine, dabrafenib, dasatinib, daunorubicin, decitabine, duocarmycin, enasidenib, epirubicin, eribulin mesylate, erlotinib, everolimus, etoposide, exatecan, fludarabine, fulvestrant, gemcitabine, hemiasterlin, histone deacetylase inhibitors (abexinostat, belinostat, entinostat, givinostat, mocetinostat, oxamflatin, panobinostat, pyroxamide, quisinostat, vorinostat, trichostatin A, tacedinaline, tucidinostat, tubastatin A), hydroxyurea, ibrutinib, idelalisib, imatinib, irinotecan, ixabepilone, lapatinib, lenalidomide, maytansine, mitomycin C, monomethyl auristatins (MMA-E, MMA-F, dolastatin 10 and dolastatinol), methotrexate, neratinib, nilotinib, niraparib, olaparib, octreotide, osimertinib, paclitaxel, palbociclib, pazopanib, pyrrolobenzodiazepine, pemetrexed, pomalidomide, ponatinib, ribociclib, rucaparib, sorafenib, tamoxifen, triazenes, trifluridine, topotecan, tubulysin, vandetanib, and combinations or derivatives thereof.

5. A **drug delivery system characterized by** being based on tetraoxanes compounds of **formula IIc, IId** and **IIe,** as described in claim 1, **wherein the tumour marker API is** selected from 4,4-difluoro-4-bora-3a,4a-diaza-s-indacene (BODIPY), cyanines, dicyanomethines, doxorubicin, fluorescein, nitrobenzofurazan, rhodamine and combinations or derivatives thereof.

6. **A process** for the preparation of a drug delivery system based 1,2,4,5-tetraoxanes with the formula I, as described in claims 2 to 5 **characterized by** comprising the following steps: 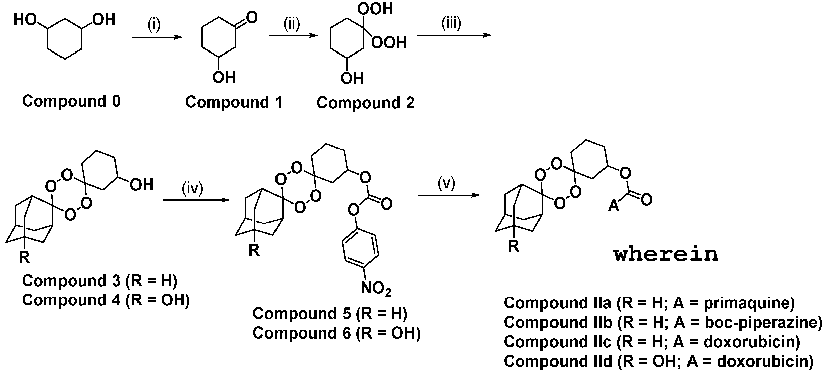
(i) Sodium dichromate dihydrate, water, sulfuric acid, react for the oxidation of 1,3-cyclohexanediol Compound **0** generating rac-3-hydroxycyclohexanone Compound **1,**
(ii) Rhenium (VII) oxide, hydrogen peroxide 50%, acetonitrile, where Compound **1** reacts with hydrogen peroxide in the presence of rhenium (VII) oxide to produce the dihydroperoxide, Compound **2,**
(iii) 2-adamantanone or 5-hydroxy-2-adamantanone, rhenium (VII) oxide, dichloromethane:acetonitrile (1:1), inert atmosphere, where dihydroperoxide Compound **2** reacts with a carbonyl reagent, employing a catalytic amount of rhenium(VII) oxide, and 2-Adamantanone (Compound **3**) and 5-hydroxy-2-adamanatone (Compound **4**) can be used as carbonyl for the formation of tetraoxanes,
(iv) 4-nitrophenyl chloroformate, *N,N-*diisopropylethylamine, 4-dimethylaminopyridine, dichloromethane, where compound **3** and compound **4** are activated with 4-nitrophenyl chloroformate together with excess amine bases (*N,N*-diisopropylethylamine and 4-dimethylaminopyridine) to generate compounds (**5**) and (**6**), respectively, and
(v) API, 1-hydroxybenzotriazole hydrate, triethylamine, *N,N*-dimethylformamide, where Compound **5** is coupled with primaquine-diphosphate to generate the compound **IIa** or with boc-piperazine to generate the compound **IIb** or two carbonates are coupled with doxorubicin to obtain the carbamates **IIc** and **IId.**

7. A process for the preparation of a drug delivery system according to claim 6, wherein:
- in step i), sodium dichromate dihydrate, water, sulfuric acid, and compound **0 are replaced by** formaldehyde, N-methylpyrrolidine, barium hydroxide octahydrate, water:methanol (5:1), and compound **7,**
- in step ii), compound **1 is replaced by** compound **8,**
- in step iii), compound **2 is replaced by** compound **9,**
- in step iv), compound **3** or **4 is replaced by** compound **10,** and
- in step v) compound **5** or **6 is replaced by** compound **11.**

8. **An intermediary compound** represented by any of the formulae below:
**Compound 2** is 3,3-dihydroperoxycyclohexan-1-ol
**Compound 3** is dispiro[adamantane-2,3'-[1,2,4,5]tetraoxane-6',1''-cyclohexan]-3''-ol
**Compound 4** is dispiro[adamantane-2,3'-[1,2,4,5]tetraoxane-6',1''-cyclohexane]-3'',5-diol
**Compound 5** is dispiro[adamantane-2,3'-[1,2,4,5]tetraoxane-6',1''-cyclohexan]-3''-yl (4-nitrophenyl) carbonate
**Compound 6** is 5-hydroxydispiro[adamantane-2,3'-[1,2,4,5]tetraoxane-6',1''-cyclohexan]-3''-yl (4-nitrophenyl) carbonate
**Compound 9** is 6,6-dihydroperoxycyclohex-1-en-1-yl)methanol
**Compound 10** is 2' '-(hydroxymethyl)dispiro[adamantane-2,3'-[1,2,4,5]tetraoxane-6',1''-cyclohexan]-2''-en-5-ol **Compound 11** is 5-hydroxydispiro[adamantane-2,3'-[1,2,4,5] tetraoxane-6',1''-cyclohexan]-2''-en-2''-yl)methyl (4-nitrophenyl) carbonate.

9. A **compound of formulae IIa, IIb, IIc, IId** and **IIe** as described in claim 1, or **a drug delivery system comprising said compounds,** as described in any of the claims 2 to 4 **characterized by being used as a medicament.**

10. **A drug delivery system** according to claim 9, wherein:
- the compounds of formula **IIa** and **IIb** as described in claim 3 are **characterized by** being used as **antimalarial drug,**
- the compounds **IIc, IId** or **IIe** as described in claim 4 are **characterized by** being used **as antitumour drug.**

11. **A compound of formulae IIc, IId** or **IIe, as described in claim 1, or a drug delivery system, comprising said compounds IIc, IId** or **IIe** as described in claim 5 are **characterized by** being used **as a tumour marker.**

## Patentansprüche

1. Eine **Zusammensetzung** der Formel I beinhaltend Enantiomere und racemische Mischungen davon, wobei: 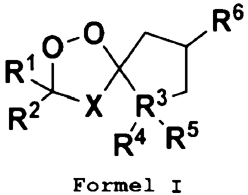
• R¹ und R² unabhängig voneinander Wasserstoff, ersetzbare oder nicht ersetzbare Alkylgruppe, ersetzbares oder nicht ersetzbares Heteroalkyl, ersetzbares oder nicht ersetzbares Cycloalkyl, ersetzbares oder nicht ersetzbares Heterocycloalkyl, ersetzbares oder nicht ersetzbares Aryl und ersetzbares oder nicht ersetzbares Heteroaryl sind,
• R¹ und R² miteinander verbunden sein können, um ersetzbares oder nicht ersetzbares Cycloalkyl und ersetzbares oder nicht ersetzbares Heteroycloalkyl zu bilden,
• X -CH₂O-, -OCH₂- oder -O-O- ist
• R³-C(R⁴)(R⁵)-, -CH(R⁴)-(R⁵)- oder -C(R⁴)=C(R⁵)- ist
• R⁴ Wasserstoff, -OH, -CH₂OH, -CH₂=O(C=O)A oder -CH₂O(C=S)A ist, wobei A unabhängig ein oder mehr APIs, Proteine und Antikörper ist,
• R⁵ und R⁶ unabhängig voneinander Wasserstoff, -OH, -O(C=O)A oder -O(C=S)A sind, wobei A unabhängig ein oder mehr APIs, Proteine und Antikörper ist,
und so eine Zusammensetzung **dadurch gekennzeichnet** ist, durch eine der folgenden Formeln dargestellt zu werden:
**die Formel IIa, wobei:**
R¹ Adamantyl ist
R² Adamantyl ist
X -O-O- ist
R³ -CH(R⁴)-CH(R⁵)- ist
R⁴ Wasserstoff ist
R⁵ (4-((7-methoxyquinolin-5-yl)amino)pentyl)-Carbamoyloxy ist
und
R⁶ Wasserstoff ist 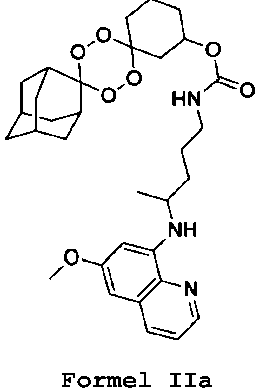 **die Formel IIb, wobei:**
R¹ Adamantyl ist
R² Adamantyl ist
X -O-O- ist
R³ -CH(R⁴)-CH(R⁵)- ist
R⁴ Wasserstoff ist
R⁵ 1-(tert-Butyl)Piperazin-1,4-Dicarboxylat ist und
R⁶ Wasserstoff ist 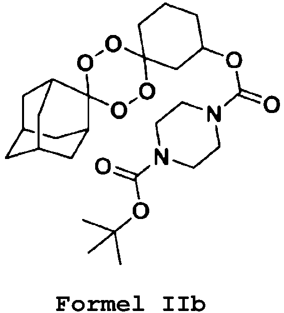 **die Formel IIc, wobei:**
R¹ Adamantyl ist
R² Adamantyl ist
X -O-O- ist
R³ -CH(R⁴)-CH(R⁵)- ist
R⁴ Wasserstoff ist
R⁵ (3-Hydroxy-2-Methyl-6-((2S,4S)-4,5,12-Trihydroxy-4-(2-Hydroxyacetyl)-10-Methoxy-6,11-Dioxo-1,2,3,4,6,11-Hexahydrotetracen-2-yl)oxy)Tetrahydro-2H- Pyran-4-yl) Carbamoyloxy ist und
R⁶ Wasserstoff ist 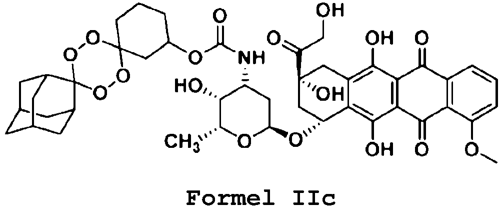 **die Formel IId, wobei:**
R¹ 5-Hydroxy-Adamantyl ist
R² 5-Hydroxy-Adamantyl ist
X -O-O- ist
R³ -CH(R⁴)-CH(R⁵)- ist
R⁴ Wasserstoff ist
R⁵ (3-Hydroxy-2-Methyl-6-(((2S,4S)-4,5,12-Trihydroxy-4-(2- Hydroxyacetyl)-10-Methoxy-6,11-Dioxo-1,2,3,4,6,11-Hexahydrotetracen-2-yl)oxy)Tetrahydro-2H-Pyran-4-yl)-Carbamoyloxy ist, und
R⁶ Wasserstoff ist 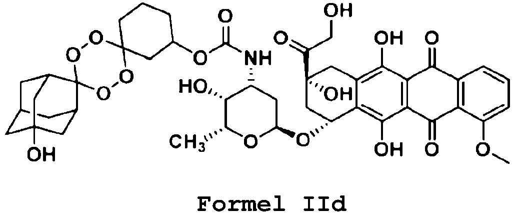 **die Formel IIe, wobei:**
R¹ 5-Hydroxy-Adamantyl ist
R² 5-Hydroxy-Adamantyl ist
X -O-O- ist
R³ -CH(R⁴)=CH(R⁵)- ist
R⁴ (3-Hydroxy-2-Methyl-6-(((1R,3R)-3,5,12-Trihydroxy-3-(2- Hydroxyacetyl)-10-Methoxy-6,11-Dioxo-1,2,3,4,6,11-Hexahydrotetracen-1-yl)oxy)Tetrahydro-2H-Pyran-4-yl)-Carbamoyloxy ist R⁵ Wasserstoff ist und
R⁶ Wasserstoff ist

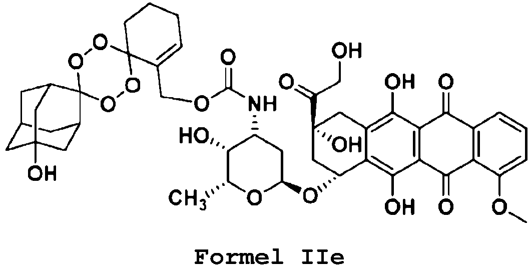

2. Ein **System zur Verabreichung von Medikamenten dadurch gekennzeichnet, dass** es auf Tetraoxanzusammensetzungen der **Formeln IIa, IIb, IIc, IId** und **IIe** basiert, wie beschrieben in Anspruch 1, wobei diese Zusammensetzungen entsprechend verändert werden, um pharmazeutische Wirkstoffe (API) freizusetzen, wobei die **Zusammensetzungen IIa oder IIb mit einem oder mehreren Antimalaria-APIs, die Zusammensetzungen IIc, IId oder IIe mit einem oder mehreren Antitumor-APIs oder die Zusammensetzungen IIc, IId oder IIe mit einem oder mehreren Tumormarker-APIs verbunden werden.**

3. Ein **System zur Verabreichung von Medikamenten** gemäß Anspruch 2 durch die **Antimalaria**-APIs gekennzeichnet, die aus Primaquin und Boc-Piperazin, Atovaquon, Chinin-Sulfat, Sulfonamiden (Sulfadoxin und Sulfamethoxypyridazin) und Falcipain-Inhibitoren (Dipeptidylvinylsulfonen und peptidomimetischen Pyrimidinnitrilen) sowie Zusammenstellungen oder Derivaten davon ausgewählt sind.

4. Ein **System zur Verabreichung von Medikamenten** gemäß Anspruch 2 durch die **Antitumor**-APIs gekennzeichnet, die aus Doxorubicin, Alectinib, Afatinib, Abirateronacetat, Azacitidin, Axitinib, Bendamustin, Brigatinib, Bosutinib, Bicalutamid, Calicheamicin, Capecitabin, Carfilzomib, Combretastatin, Ceritinib, Cladribin, Clofarabin, Cobimetinib, Crizotinib, Cryptophycin, Cytarabin, Dabrafenib, Dasatinib, Daunorubicin, Decitabin, Duocarmycin, Enasidenib, Epirubicin, Eribulin Mesylat, Erlotinib, Everolimus, Etoposid, Exatecan, Fludarabin, Fulvestrant, Gemcitabin, Hemiasterlin, Histon-Deacetylase-Inhibitoren (Abexinostat, Belinostat, Entinostat, Givinostat, Mocetinostat, Oxamflatin, Panobinostat, Pyroxamid, Quisinostat, Vorinostat, Trichostatin A, Tacedinalin, Tucidinostat, Tubastatin A), Hydroxyharnstoff, Ibrutinib, Idelalisib, Imatinib, Irinotecan, Ixabepilon, Lapatinib, Lenalidomid, Maytansin, Mitomycin C, Monomethyl-Auristatine (MMA-E, MMA-F, Dolastatin 10 und Dolastatinol), Methotrexat, Neratinib, Nilotinib, Niraparib, Olaparib, Octreotid, Osimertinib, Paclitaxel, Palbociclib, Pazopanib, Pyrrolobenzodiazepin, Pemetrexed, Pomalidomid, Ponatinib, Ribociclib, Rucaparib, Sorafenib, Tamoxifen, Triazen, Trifluridin, Topotecan, Tubulysin, Vandetanib und Zusammenstellungen oder Derivaten davon ausgewählt sind.

5. Ein **System zur Verabreichung von Medikamenten dadurch gekennzeichnet, dass** es auf Tetraoxanzusammensetzungen der **Formeln IIc, IId** und **IIe** basiert, wie beschrieben in Anspruch 1, **wobei der Tumormarker-API** aus 4,4-Difluor-4-bora-3a,4a-diaza-s-indacen (BODIPY), Cyaninen, Dicyanomethinen, Doxorubicin, Fluorescein, Nitrobenzofurazan, Rhodamin und Zusammenstellungen oder Derivaten davon ausgewählt ist.

6. Ein **Verfahren** zur Zubereitung eines Systems zur Verabreichung von Medikamenten auf Basis von 1,2,4,5-Tetraoxanen der Formel I, wie beschrieben in den Ansprüchen 2 bis 5, **gekennzeichnet durch** die folgenden Schritte: 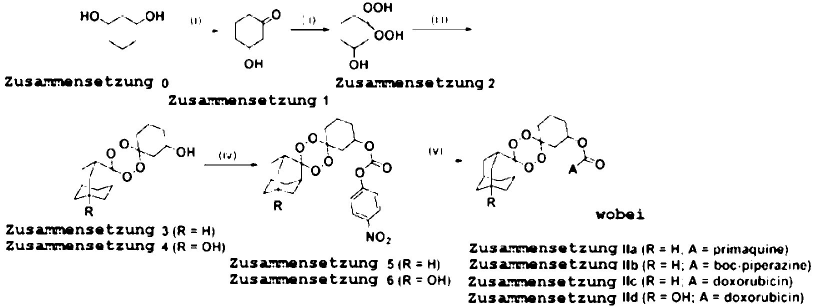
(i) Natriumdichromat-Dihydrat, Wasser, Schwefelsäure, reagieren zur Oxidation von 1,3-Cyclohexandiol Zusammensetzung **0** unter Bildung von rac-3-Hydroxycyclohexanon, Zusammensetzung **1,**
(ii) Rhenium(VII)-oxid, Wasserstoffperoxid 50 %, Acetonitril, wobei Zusammensetzung **1** in Gegenwart von Rhenium(VII)-oxid mit Wasserstoffperoxid reagiert, um das Dihydroperoxid zu bilden, Zusammensetzung **2,**
(iii) 2-Adamantanone oder 5-Hydroxy-2-adamantanone, Rhenium(VII)-oxid, Dichlormethan:Acetonitril (1:1), träge Atmosphäre, wobei die Dihydroperoxid Zusammensetzung **2** mit einem Carbonylreagenz unter Verwendung einer katalytischen Menge an Rhenium(VII)-oxid reagiert, und 2-Adamantanone (Zusammensetzung **3**) und 5-Hydroxy-2-adamanatone (Zusammensetzung **4**) können als Carbonyl zur Bildung von Tetraoxanen verwendet werden,
(iv) 4-Nitrophenylchlorformiat, *N,N*-Diisopropylethylamin, 4-Dimethylaminopyridin, Dichlormethan, wobei Zusammensetzung **3** und Zusammensetzung **4** mit 4-Nitrophenylchlorformiat zusammen mit überschüssigen Aminbasen (*N,N*-Diisopropylethylamin und 4-Dimethylaminopyridin) aktiviert werden, um die Zusammensetzungen (**5**) bzw. (**6**) zu erzeugen, und
(v) API, 1-Hydroxybenzotriazolhydrat, Triethylamin, *N,N-*Dimethylformamid, wobei Zusammensetzung **5** mit Primaquindiphosphat verbunden wird, um die Zusammensetzung **IIa** zu erzeugen, oder mit Boc-Piperazin, um die Zusammensetzung **IIb** zu erzeugen, oder zwei Carbonate mit Doxorubicin verbunden werden, um die Carbamate **IIc** und **IId** zu erhalten.

7. Ein Verfahren zur Zubereitung eines Systems zur Verabreichung von Medikamenten gemäß Anspruch 6, wobei:
- in Schritt i), Natriumdichromat-Dihydrat, Wasser, Schwefelsäure und Zusammensetzung **0 werden ersetzt durch** Formaldehyd, N-Methylpyrrolidin, Bariumbicarbonat-Octahydrat, Wasser:Methanol (5:1) und Zusammensetzung **7,**
- in Schritt ii), Zusammensetzung **1 wird ersetzt durch** Zusammensetzung **8,**
- in Schritt iii), Zusammensetzung **2 wird ersetzt durch** Zusammensetzung **9,**
- in Schritt iv), Zusammensetzung **3** oder **4 wird ersetzt durch** Zusammensetzung **10,** und
- in Schritt v), Zusammensetzung **5** oder 6 **wird ersetzt durch** Zusammensetzung **11.**

8. Eine **Zwischenzusammensetzung** dargestellt durch einer der untenstehenden Formeln:
**Zusammensetzung 2** ist 3,3-Dihydroperoxycyclohexan-1-ol
**Zusammensetzung 3** ist Dispiro[Adamantan-2,3'-[1,2,4,5]tetraoxan-6',1''-cyclohexan]-3''-ol
**Zusammensetzung 4** ist Dispiro[Adamantan-2,3'-[1,2,4,5]tetraoxan-6',1''-cyclohexan]-3'',5-diol
**Zusammensetzung 5** ist Dispiro[Adamantan-2,3'-[1,2,4,5]tetraoxan-6',1''-cyclohexan]-3''-yl(4-nitrophenyl)carbonat
**Zusammensetzung 6** ist 5-Hydroxydispiro[adamantan-2,3'-[1,2,4,5]tetraoxan-6',1''-cyclohexan]-3''-yl(4-nitrophenyl)carbonat
**Zusammensetzung 9** ist 6,6-Dihydroperoxycyclohex-1-en-1-yl)methanol
**Zusammensetzung 10** ist 2''-(Hydroxymethyl)dispiro[adamantan-2,3'-[1,2,4,5]tetraoxan-6',1''-cyclohexan]-2''-en-5-ol
**Zusammensetzung 11** ist 5-Hydroxydispiro[adamantan-2,3'-[1,2,4,5]tetraoxan-6',1''-cyclohexan]-2''-en-2''-yl)methyl(4-nitrophenyl)carbonat.

9. Eine **Zusammensetzung der Formeln IIa, IIb, IIc, IId** und **IIe** wie beschrieben in Anspruch 1 oder **ein System zur Verabreichung von Medikamenten umfassend genannte Zusammensetzungen** wie beschrieben in einer Ansprüchen 2 bis 4 **dadurch gekennzeichnet, dass** sie **als Medikament verwendet** werden.

10. **Ein System zur Verabreichung von Medikamenten** gemäß Anspruch 9, wobei:
- die Zusammensetzungen der Formeln **IIa,** und **IIb** wie beschrieben in Anspruch 3 **dadurch gekennzeichnet, dass** sie als **Antimalariamedikament** verwendet werden,
- die Zusammensetzungen der Formeln **IIc, IId** und **IIe** wie beschrieben in Anspruch 4 **dadurch gekennzeichnet, dass** sie als **Antitumormedikament** verwendet werden.

11. **Eine Zusammensetzung der Formeln IIc, IId** und **IIe** wie beschrieben in Anspruch 1 oder **ein System zur Verabreichung von Medikamenten umfassend genannte Zusammensetzungen IIc, IId** und **IIe** wie beschrieben in Anspruch 5 **dadurch gekennzeichnet, dass** sie **als Tumormarker** verwendet werden.

## Revendications

1. **Composé** de la formule I, y compris des énantiomères et des mélanges racémiques de celle-ci, où : 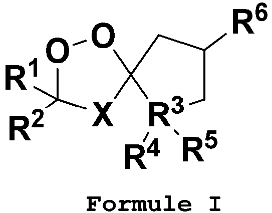
• R¹ et R² sont, indépendamment, de l'hydrogène, de l'alkyle substitué ou non substitué, de l'hétéroalkyle substitué ou non substitué, du cycloalkyle substitué ou non substitué, de l'hétérocycloalkyle substitué ou non substitué, de l'aryle substitué ou non substitué et de l'hétéroaryle substitué ou non substitué,
• R¹ et R² peuvent être joints afin de former un cycloalkyle substitué ou non substitué et un hétérocycloalkyle substitué ou non substitué,
• X est -CH₂O-, -OCH₂- ou -O-O-
• R³ est -C(R⁴)(R⁵)-, -CH(R⁴)-CH(R⁵)- ou -C(R⁴)=C(R⁵)-
• R⁴ est de l'hydrogène, -OH, -CH₂OH, -CH₂O(C=O)A ou - CH₂O(C=S)A, où A est, indépendamment, une ou plusieurs IPA, protéines et anticorps,
• R⁵ et R⁶ sont, indépendamment, de l'hydrogène, -OH, - O(C=O)A ou -O(C=S)A, où A est, indépendamment, une ou plusieurs IPA, protéines ou anticorps,
**et un tel composé est caractérisé par le fait qu'il est représenté par l'une des formules suivantes, ci-dessous** : **Formule IIa, où**
**R¹** est de l'adamantyle
**R²** est de l'adamantyle
**X** est -O-O-
**R³** est -CH(R⁴)-CH(R⁵)-
**R⁴** est de l'hydrogène
**R⁵** est (4-((7-méthoxyquinoline-5-yl)amino)pentyle) carbamoyloxy et
**R⁶** est de l'hydrogène 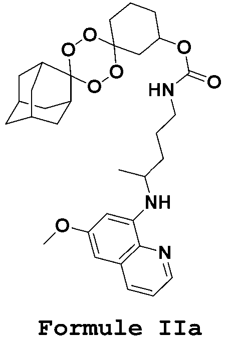 **Formule IIb, où**
**R¹** est de l'adamantyle
**R²** est de l'adamantyle
**X** est -O-O-
**R³** est -CH(R⁴)-CH(R⁵)-
**R⁴** est de l'hydrogène
**R⁵** est 1-(tert-butyle) pipérazine-1,4-dicarboxylate et
**R⁶** est de l'hydrogène 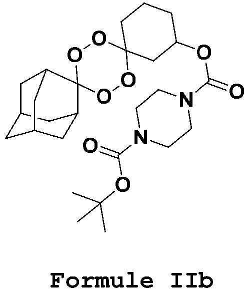 **Formule IIc, où**
**R¹** est de l'adamantyle
**R²** est de l'adamantyle
**X** est -O-O-
**R³** est -CH(R⁴)-CH(R⁵)-
**R⁴** est de l'hydrogène
**R⁵** est (3-hydroxy-2-méthyle-6-((2S,4S)-4,5,12-trihydroxy-4-(2-hydroxyacétyle)-10-méthoxy-6,11-dioxo-1,2,3,4,6,11-hexahydrotétracène-2-yl)oxy)tetrahydro-2H-pyrane-4-yl)carbamoyloxy et
**R⁶** est de l'hydrogène 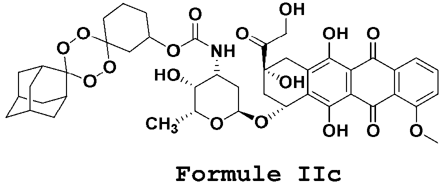 **Formule IId, où**
**R¹** est 5-hydroxy adamantyle
**R²** est 5-hydroxy adamantyle
**X** est -O-O-
**R³** est -CH(R⁴)-CH(R⁵)-
**R⁴** est de l'hydrogène
**R⁵** est (3-hydroxy-2-méthyle-6-(((2S,4S)-4,5,12-trihydroxy-4-(2-hydroxyacétyle)-10-methoxy-6,11-dioxo-1,2,3,4,6,11-hexahydrotétracène-2-yl)oxy)tétrahydro-2H-pyrane-4-yl) carbamoyloxy et
**R⁶** est de l'hydrogène 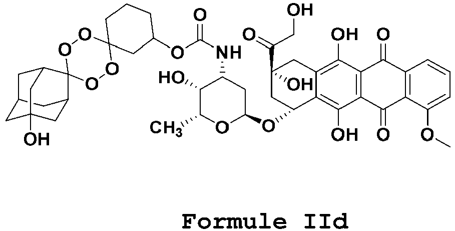 **Formule IIe, où**
**R¹** est 5-hydroxy adamantyle
**R²** est 5-hydroxy adamantyle
**X** est -O-O-
**R³** est -C(R⁴)=C(R⁵)-
**R⁴** est (3-hydroxy-2-méthyle-6-(((1R,3R)-3,5,12-trihydroxy-3-(2-hydroxyacétyle)-10-methoxy-6,11-dioxo-1,2,3,4,6,11-hexahydrotétracène-1-yl)oxy)tetrahydro-2H-pyrane-4-yl)carbamoyloxy
**R⁵** est de l'hydrogène et
**R⁶** est de l'hydrogène

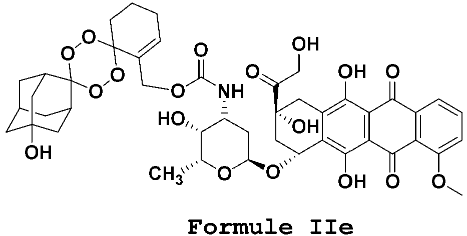

2. **Système de délivrance de médicaments caractérisé par le fait qu'**il se fonde sur des composés de tétraoxanes des **formules IIa, IIb, IIc, IId** et **IIe,** tels que décrits dans la revendication 1, ces composés étant modifiés de manière appropriée pour libérer des ingrédients pharmaceutiques actifs (IPA), où **les composés IIa ou IIb sont couplés avec un ou plusieurs IPA antipaludiques, les composés IIc, IId ou IIe sont couplés avec un ou plusieurs IPA antitumoraux ou les composés IIc, IId ou IIe sont couplés avec un ou plusieurs IPA marqueurs tumoraux.**

3. **Système de délivrance de médicaments** selon la revendication 2, **caractérisé par le fait que** les IPA **antipaludiques** sont sélectionnés depuis la primaquine et la boc-pipérazine, l'atovaquone, le sulfate de quinine, des sulfonamides (la sulfadoxine et la sulfaméthoxypyridazine) et des inhibiteurs de falcipaines (les sulfones vinyles dipeptidyl et les nitriles pyrimidines peptidomimétiques) et des combinaisons ou dérivés de ceux-ci.

4. **Système de délivrance de médicaments** selon la revendication 2 **caractérisée par le fait que** les IPA **antitumoraux** sont sélectionnés depuis la doxorubicine, l'alectinib, l'afatinib, l'acétate abiratérone, l'azacitidine, l'axitinib, la bendamustine, le brigatinib, le bosutinib, la bicalutamide, la calichéamicine, la capécitabine, le carfilzomib, la combrétastatine, le cérétinib, la cladribine, la clofarabine, le cobimétinib, le crizotinib, la cryptophycine, la cytarabine, le dabrafénib, le dasatinib, la daunorubicine, la décitabine, la duocarmycine, le enasidénib, l'épirubicine, l'éribuline mésylate, l'erlotinib, l'évérolimus, l'étoposide, l'exatécan, la fludarabine, le fulvestrant, la gemcitabine, l'hémiasterline, les inhibiteurs d'histone désacétylase (abexinostat, belinostat, entinostat, givinostat, mocetinostat, oxamflatin, panobinostat, pyroxamide, quisinostat, vorinostat, trichostatine A, tacedinaline, tucidinostat, tubastatine A), l'hydroxyurée, l'ibrutinib, l'idélalisib, l'imatinib, l'irinotécan, l'ixabépilone, le lapatinib, le lénalidomide, la maytansine, la mitomycine C, les monométhyl auristatines (MMA-E, MMA-F, dolastatine 10 et dolastatinol), le méthotrexate, le nératinib, le nilotinib, le niraparib, l'olaparib, l'octréotide, l'osimertinib, le paclitaxel, le palbociclib, le pazopanib, la pyrrolobenzodiazépine, le pemetrexed, le pomalidomide, le ponatinib, le ribociclib, le rucaparib, le sorafénib, le tamoxifène, les triazenes, la trifluridine, le topotécan, la tubulysine, le vandétanib et des combinaisons ou des dérivés de ceux-ci.

5. **Système de délivrance de médicaments** caractérisé comme étant la base de composés de tétraoxanes des **formules IIc, IId** et **IIe,** tel que décrit dans la revendication 1, où **l'IPA de marqueur tumoral** est sélectionné depuis 4,4-difluoro-4-bora-3a,4a-diaza-s-indacène (BODIPY), des cyanines, des dicyanométhines, de la doxorubicine, de la fluorescéine, du nitrobenzofurazane, de la rhodamine et des combinaisons ou des dérivés de ceux-ci.

6. **Processus** de préparation d'un système de délivrance de médicaments fondé sur les 1,2,4,5-tétraoxanes avec la formule I, tel que décrit dans les revendications 2 à 5, **caractérisé par le fait qu'**il comprend les étapes suivantes : 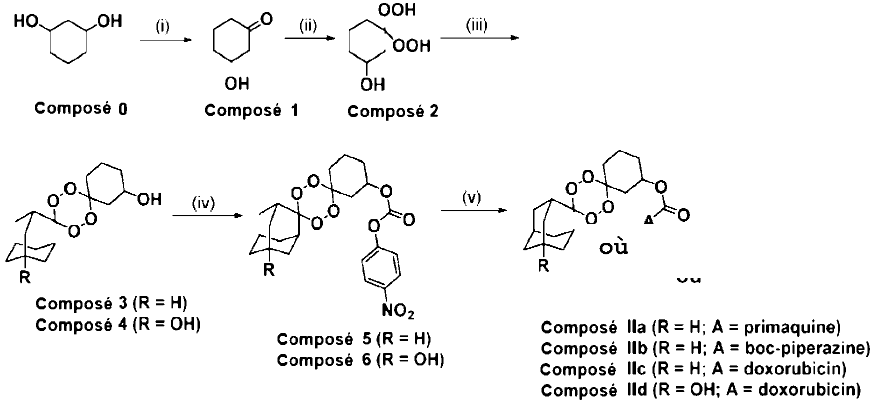
(i) Le dihydrate de dichromate de sodium, l'eau, l'acide sulfurique réagissent à l'oxydation du Composé 0 de 1,3-cyclohexanediol, générant le Composé **1** de rac-3-hydroxycyclohexanone,
(ii) L'oxyde de rhénium(VII), le péroxyde d'hydrogène à 50 %, l'acétonitrile, où le Composé 1 réagit avec le péroxyde d'hydrogène en présence de l'oxyde rhénium (VII) afin de produire le di-hydroperoxyde, le Composé **2,**
(iii) Le 2-adamantanone ou le 5-hydroxy-2-adamantanone, l'oxyde de rhénium (VII), le dichlorométhane:acétonitrile (1:1), l'atmosphère inerte, où le Composé **2** de di-hydroperoxyde réagit au réactif carbonyle, en employant une quantité catalytique d'oxyde de rhénium (VII), et le 2-Adamantanone (Composé **3**) ainsi que le 5-hydroxy-2-adamanatone (Composé **4**) peuvent être utilisés comme carbonyle pour la formation de tétraoxanes,
(iv) Le chloroformiate de 4-nitrophényle, la N,N-diisopropyléthylamine, la 4-diméthylaminopyridine, le dichlorométhane, où le composé **3** et le composé **4** sont activés avec le chloroformiate de 4-nitrophényle, ensemble, avec des bases d'amine en excès (N,N-diisopropyléthylamine et 4-diméthylaminopyridine) afin de générer des composés (**5**) et (**6**), respectivement, et
(v) L'IPA, l'hydrate de 1-hydroxybenzotriazole, la triéthylamine, le *N,N*-diméthylformamide, où le Composé **5** est couplé avec la primaquine-diphosphate afin de générer le composé **IIa** ou avec le boc-pipérazine afin de générer le composé **IIb** ou deux carbonates sont couplés avec la doxorubicine pour obtenir les carbamates **IIc** et **IId.**

7. Processus de préparation d'un système de délivrance de médicaments selon la revendication 6, où :
- à l'étape i), le dihydrate de dichromate de sodium, l'eau, l'acide sulfurique et le composé **0 sont remplacés** par le formaldéhyde, la N-méthylpyrrolidone, l'octahydrate d'hydroxyde de baryum, l'eau:méthanol (5:1), et le composé **7,**
- à l'étape ii), le composé **1 est remplacé par** le composé **8,**
- à l'étape iii), le composé **2 est remplacé par** le composé **9,**
- à l'étape iv), le composé **3** ou **4 est remplacé par** le composé 1**0**, et
- à l'étape v), le composé **5** ou **6 est remplacé par** le composé **11.**

8. **Composé intermédiaire** représenté par une quelconque formule ci-dessous :
Le **composé 2** est du 3,3-dihydroperoxycyclohexane-1-ol
Le **composé 3** est du dispiro[adamantane-2,3'-[1,2,4,5]tétraoxane-6',1''-cyclohexane]-3''-ol
Le **composé 4** est du dispiro[adamantane-2,3'-[1,2,4,5]tétraoxane-6',1''-cyclohexane]-3'',5-diol
Le **composé 5** est du dispiro[adamantane-2,3'-[1,2,4,5]tétraoxane-6',1''-cyclohexane]-3''-yl (4-nitrophényle) carbonate
Le **composé 6** est du 5-hydroxydispiro[adamantane-2,3'-[1,2,4,5]tétraoxane-6',1''-cyclohexane]-3''-yl(4-nitrophényle) carbonate
Le **composé 9** est 6,6-dihydroperoxycyclohexane-1-en-1-yl)méthanol
Le **composé 10** est du 2''-(hydroxyméthyle)dispiro[adamantane-2,3'-[1,2,4,5]tétraoxane-6',1''-cyclohexane]-2''-en-5-ol
Le **composé 11** est du 5-hydroxydispiro[adamantane-2,3'-[1,2,4,5]
tétraoxane-6',1''-cyclohexane]-2''-en-2''-yl)méthyle (4-nitrophényle) carbonate.

9. Composé des formules **IIa, IIb, IIc, IId** et **IIe,** tel que décrit dans la revendication 1 ou **un système de délivrance de médicaments comprenant lesdits composés,** tels que décrits dans une quelconque revendication entre 2 et 4, **caractérisé par le fait qu'ils sont utilisés en tant que médicament.**

10. **Système de délivrance de médicaments** selon la revendication 9, où :
- les composés des formules **IIa** et **IIb** tels que décrits dans la revendication 3, sont **caractérisés par le fait qu'**ils sont utilisés en tant que **médicament antipaludique,**
- les composés des formules **IIc, IId** et **IIe** tels que décrits dans la revendication 4 sont **caractérisés par le fait qu'**ils sont utilisés en tant que **médicament antitumoral.**

11. **Composé des formules IIc, IId** ou **IIe, tel que décrit dans la revendication 1 ou système de délivrance de médicament, comprenant lesdits composés IIc, IId ou IIe,** tels que décrits dans la revendication 5, sont caractérisés comme étant utilisés **en tant que marqueur tumoral.**
